(19) Europäisches Patentamt
European Patent Office
Office européen des brevets

(11) Veröffentlichungsnummer: **0 198 264**
**A2**

(12) # EUROPÄISCHE PATENTANMELDUNG

(21) Anmeldenummer: **86103814.9**

(22) Anmeldetag: **20.03.86**

(51) Int. Cl.⁴: **C07D 401/06**, A01N 43/50, C07D 215/00, C07D 219/00

(30) Priorität: **01.04.85 JP 66636/85**

(43) Veröffentlichungstag der Anmeldung: **22.10.86 Patentblatt 86/43**

(84) Benannte Vertragsstaaten: **AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kurahashi, Yoshio**
**47-15, Oya-machi**
**Hachioji-shi Tokyo(JP)**
Erfinder: **Shiokawa, Kozo**
**210-6, Shukugawara Tama-ku**
**Kawasaki-shi Tokyo(JP)**
Erfinder: **Goto, Toshio**
**3454-21, Honmachida**
**Machida-shi Tokyo(JP)**
Erfinder: **Kagabu, Shinzo**
**432-131-105, Terada-machi**
**Hachioji-shi Tokyo(JP)**
Erfinder: **Kamochi, Atsumi**
**2-24-10, Higashi-Toyoda**
**Hino-shi Tokyo(JP)**
Erfinder: **Moriya, Koichi**
**39-15, Namiki-cho**
**Hachioji-shi Tokyo(JP)**
Erfinder: **Hayakawa, Hidenori**
**3-3-19, Midori-cho**
**Musashino-shi Tokyo(JP)**

(74) Vertreter: **Ernst, Hilmar, Dr. et al**
**Bayer AG Konzernverwaltung RP**
**Patentabteilung**
**D-5090 Leverkusen, Bayerwerk(DE)**

(54) **Carbamoylimidazole.**

(57) Offenbart werden neue Carbamoylimidazole der Formel (I)

EP 0 198 264 A2

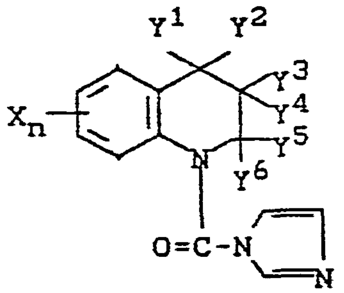

(I)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, halogensubstituiertes Niederalkylsulfonyl, halogensubstituiertes Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$, und $Y^6$ jeweils Wasserstoff, Niederalkyl, Halogen oder Phenyl sind, wobei $Y^2$ und $Y^3$ zusammen genommen eine Einfachbindung bilden können, so daß in dem heterocyclischen Ring eine Doppelbindung gebildet wird, oder $Y^4$ und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind,

sowie die Verwendung der neuen Verbindungen als Herbizide und Fungizide für Landwirtschaft und Gartenbau.

Neu sind auch Zwischenprodukte der Formel -(IV)

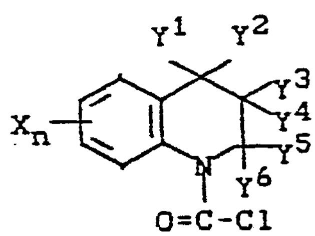

# Carbamoylimidazole

Die vorliegende Erfindung betrifft neue Carbamoylimidazole, Verfahren zu ihrer Herstellung und ihre Verwendung als Herbizide und Fungizide für Landwirtschaft und Gartenbau sowie neue Zwischenprodukte für ihre Herstellung.

Es wurde bereits offenbart, daß ein 2,4,5-Tribromoimidazol-Derivat insektizide und herbizide Aktivitäten besitzt (siehe die JP-Patentveröffentlichung 17 748/1968).

Es wurden neue Carbamoylimidazole der Formel (I),

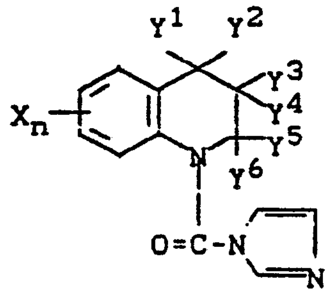

(I)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, halogensubstituiertes Niederalkylsulfonyl, halogensubstituiertes Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$, und $Y^6$ jeweils Wasserstoff, Niederalkyl, Halogen oder Phenyl sind,

wobei $Y^2$ und $Y^3$ zusammen genommen eine Einfachbindung bilden können, so daß in dem heterocyclischen Ring eine Doppelbindung gebildet wird, oder $Y^4$ und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind,

gefunden.

In den Definitionenen steht der Begriff "Nieder" jeweils für 1 bis 6 Kohlenstoff-Atome.

Die Verbindungen der Formel (I)

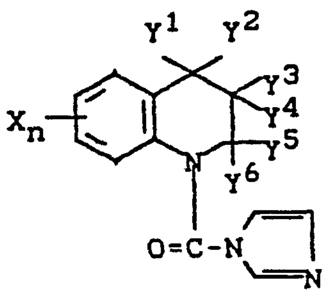

(I)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$ und $Y^6$ jeweils Wasserstoff, Niederalkyl, Halogen oder Phenyl sind,

wobei $Y^2$ und $Y^3$ zusammen genommen eine Einfachbindung bilden können, so daß in dem heterocyclischen Ring eine Doppelbindung gebildet wird, oder $Y^4$ und $Y^5$ zusammen genommen eine

Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind,

werden durch ein Verfahren erhalten, in dem

(a) die Verbindungen der Formel (II)

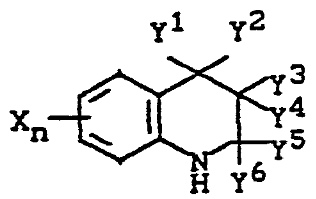

(II)

in der X, n, $Y^1$, $Y^2$, $Y^3$, $Y^4$, $Y^5$ und $Y^6$ die im Vorstehenden angegebenen Bedeutungen haben, mit N,N'-Carbonyldiimidazol der Formel (III)

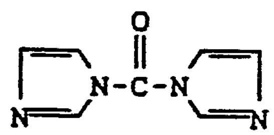

(III)

umgesetzt werden, gegebenenfalls in Gegenwart eines inerten Lösungsmittels,

oder in dem

(b) die Verbindungen der Formel (IV)

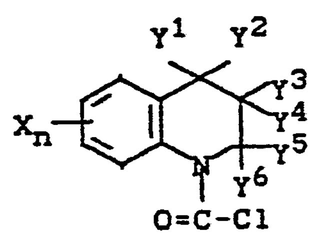

(IV)

in der X, n, $Y^1$, $Y^2$, $Y^3$, $Y^4$, $Y^5$ und $Y^6$ die im Vorstehenden angegebenen Bedeutungen haben, mit Imidazol der Formel (V)

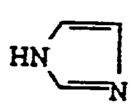

(V)

umgesetzt werden, gegebenenfalls in Gegenwart eines inerten Lösungsmittels und/oder in Gegenwart eines säurebindenden Mittels.

Schließlich wurde gefunden, daß die neuen Carbamoylimidazole hervorragende herbizide Wirkungen und auch fungizide Wirkungen in Landwirtschaft und Gartenbau zeigen.

Weiterhin gefunden wurden neue Verbindungen der Formel (IV), die Zwischenprodukte in dem Verfahren zur Herstellung der Verbindungen der Formel (I) sind.

Die Verbindungen der Formel (IV) werden erhalten, wenn die oben bezeichneten Verbindungen der Formel (II) mit Phosgen oder mit Trichloromethylchlorameisensäureester umgesetzt werden.

Überraschenderweise zeigen die erfindungsgemäßen Carbamoylimidazole gegenüber bekannten Verbindungen des Standes der Technik eine im wesentlichen stark überlegene herbizide Wirkung, insbesondere eine selektive herbizide Wirkung gegen Reisfeld-Unkräuter und auch eine fungizide Wirkung auf dem Gebiet von Landwirtschaft und Gartenbau.

In den oben angeführten Definitonen der Formeln (I), (II), und (IV) steht der Begriff "Nieder" jeweils für 1 bis 6 Kohlenstoff-Atome.

Die Formel (I) gibt eine allgemeine Definition der Carbamoylimidazole gemäß der Erfindung. Bevorzugte Verbindungen der Formel (I) sind diejenigen, in denen

X Fluor, Chlor, Brom, Alkyl mit 1 bis 4 Kohlenstoff-Atomen, Alkoxy mit 1 bis 4 Kohlenstoff-Atomen, Alkylthio mit 1 bis 4 Kohlenstoff-Atomen, fluor-und/oder chlorsubstituiertes Alkyl mit 1 bis 4 Kohlenstoff-Atomen, fluor-und/oder chlorsubstituiertes Alkoxy mit 1 bis 4 Kohlenstoff-Atomen, fluor-und/oder chlorsubstituiertes Alkylthio mit 1 bis 4 Kohlenstoff-Atomen, Nitro, Cyano, Alkylsulfonyl mit 1 bis 4 Kohlenstoff-Atomen, halogensubstituiertes Alkylsulfonyl mit 1 bis 4 Kohlenstoff-Atomen, Alkylsulfinyl mit 1 bis 4 Kohlenstoff-Atomen, Phenyl, Phenoxy, oder Phenylthio ist,

n 0, 1 oder 2 ist und

$Y^1$, $Y^2$, $Y^3$, $Y^4$, $Y^5$ und $Y^6$ jeweils Wasserstoff, Alkyl mit 1 bis 4 Kohlenstoff-Atomen, Chlor oder Phenyl sind,

wobei $Y^2$ und $Y^3$ zusammen genommen eine Einfachbindung bilden können, so daß eine Doppelbindung in dem heterocyclischen Ring gebildet wird, und $Y^4$ und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind.

Besonders bevorzugte Verbindungen der allgemeinen Formel (I) sind diejenigen, in denen

X Fluor, Chlor, Methyl, Ethyl, Propyl, Isopropyl, Methoxy, Ethoxy, Methylthio, Ethylthio, Trifluoromethyl, Trifluoromethoxy, Trifluoromethylthio oder Trifluoromethylsulfonyl sind,

n 0, 1 oder 2 ist,

$Y^1$, $Y^2$ und $Y^3$ jeweils Wasserstoff sind und

$Y^4$, $Y^5$ und $Y^6$ jeweils Wasserstoff, Methyl oder Phenyl sind, mit der Maßgabe, daß $Y^1$, $Y^2$, $Y^3$, $Y^4$, $Y^5$ und $Y^6$ nicht alle gleichzeitig Wasserstoff sind.

Eine andere Gruppe besonders bevorzugter Verbindungen der Formel (I) sind diejenigen, in denen

n 0, 1 oder 2 ist,

$Y^1$, $Y^2$, $Y^3$ und $Y^6$ jeweils Wasserstoff sind und

$Y^4$ und $Y^5$ zusammen eine Tetramethylen-Gruppe bilden, und

X die oben als besonders bevorzugt angegebenen Bedeutungen hat.

Am meisten bevorzugte Verbindungen der allgemeinen Formel (I) sind diejenigen, in denen X, $Y^1$, $Y^2$, $Y^3$, $Y^4$, $Y^5$ und $Y^6$ die oben als besonders bevorzugt angegebenen Bedeutungen haben, n 2 ist und zwei Substituenten X in der 7-Stellung und der 8-Stellung gebunden sind.

Spezielle Beispiele für die Verbindungen der allgemeinen Formel (I) der vorliegenden Erfindung umfassen insbesondere die folgenden Verbindungen:

[2,7,8-Trimethyl-1(H),2,3,4-tetrahydrochinolin-1-ylcarbonyl]imidazol,

[7,8-Dichloro-3-methyl-1(H),2,3,4-tetrahydrochinolin-1-ylcarbonyl]imidazol,

[2,2,7,8-Tetramethyl-1(H),2,3,4-tetrahydrochinolin-1-ylcarbonyl]imidazol,

[2,2,7,8-Tetramethyl-1(H),2-dihydrochinolin-1-ylcarbonyl]imidazol,

[8-Chloro-2,2,7-trimethyl-1(H),2,3,4-tetrahydrochinolin-1-ylcarbonyl]imidazol,

[7-Chloro-2,2,8-trimethyl-1(H),2,3,4-tetrahydrochinolin-1-ylcarbonyl]imidazol,

[7-Fluoro-2,8-dimethyl-1(H),2,3,4-tetrahydrochinolin-1-ylcarbonyl]imidazol und

[2,2,4,7,8-Pentamethyl-1(H),2-dihydrochinolin-1-ylcarbonyl]imidazol.

Wenn beispielsweise 2,2,7,8-Tetramethyl-1,2,3,4-tetrahydrochinolin und N,N'-Carbonyldiimidazol als Ausgangssubstanzen eingesetzt werden, läßt sich der Ablauf der Reaktion in dem erfindungsgemäßen Verfahren (a) durch die folgende Gleichung darstellen:

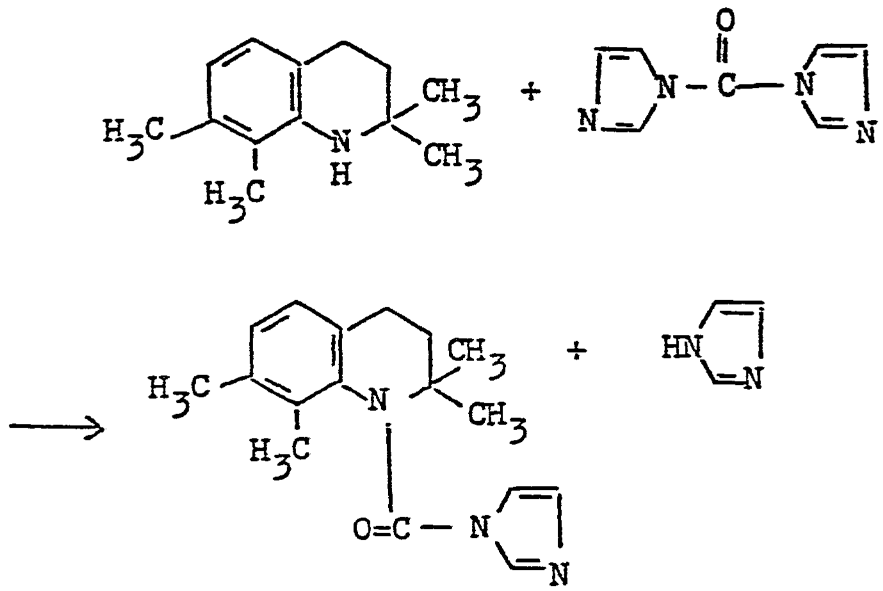

Wenn beispielsweise 2,7,8-Trimethyl-1,2,3,4-tetrahydrochinolin und Imidazol als Ausgangssubstanzen eingesetzt werden, läßt sich der Ablauf der Reaktion in dem erfindungsgemäßen Verfahren (b) durch die folgende Gleichung darstellen:

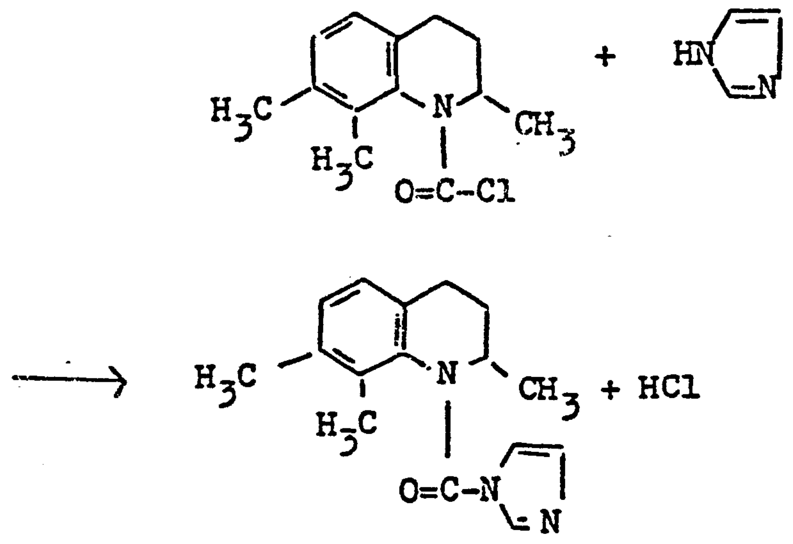

In dem Verfahren (a) bezeichnet die Formel -(II) Verbindungen, in denen X, n, $Y^1$, $Y^2$, $Y^3$, $Y^4$, $Y^5$ und $Y^6$ die oben angegebenen Bedeutungen haben, und vorzugsweise entsprechen X, n, $Y^1$, $Y^2$, $Y^3$, $Y^4$, $Y^5$ und $Y^6$ den oben als bevorzugt angegebenen Bedeutungen.

Zu den Verbindungen der Formel (II) zählen solche, die neu sind, sowie auch solche, die bereits vor dem Prioritätsdatum der vorliegenden Anmeldung bekannt waren. Als spezielle Beispiele seien die folgenden Verbindungen aufgeführt:

8-Chloro-2-methyl-1,2,3,4-tetrahydrochinolin,

8-Fluoro-2-methyl-1,2,3,4-tetrahydrochinolin,

2,8-Dimethyl-1,2,3,4-tetrahydrochinolin,

8-Ethoxy-2-methyl-1,2,3,4-tetrahydrochinolin,

2,7,8-Trimethyl-1,2,3,4-tetrahydrochinolin,

7,8-Dichloro-3-methyl-1,2,3,4-tetrahydrochinolin,

6,8-Dichloro-4-methyl-1,2,3,4-tetrahydrochinolin,

7,8-Dichloro-4-methyl-1,2,3,4-tetrahydrochinolin,

2,2,7,8-Tetramethyl-1,2,3,4-tetrahydrochinolin,

2,4,7,8-Tetramethyl-1,2,3,4-tetrahydrochinolin,

2,2,4,7,8-Pentamethyl-1,2,3,4-tetrahydrochinolin,

7,8-Dichloro-1,2-dihydrochinolin,

2,2,7,8-Tetramethyl-1,2-dihydrochinolin,

2,2,4,7,8-Pentamethyl-1,2-dihydrochinolin,

1,2,3,4,4a,9,9a,10-Octahydroacridin,

5-Chloro-1,2,3,4,4a,9,9a,10-octahydroacridin,

5-Methyl-1,2,3,4,4a,9,9a,10-octahydroacridin,

5,6-Dimethyl-1,2,3,4,4a,9,9a,10-octahydroacridin,

2-Methyl-1,2,3,4-tetrahydrochinolin,

3-Methyl-1,2,3,4-tetrahydrochinolin,

4-Methyl-1,2,3,4-tetrahydrochinolin,

2-Ethyl-1,2,3,4-tetrahydrochinolin,

2-Propyl-1,2,3,4-tetrahydrochinolin,

2-Isopropyl-1,2,3,4-tetrahydrochinolin,

2,2-Dimethyl-1,2,3,4-tetrahydrochinolin,

2,2-Diethyl-1,2,3,4-tetrahydrochinolin,

2,2,4-Trimethyl-1,2,3,4-tetrahydrochinolin,

8-Chloro-2-ethyl-1,2,3,4-tetrahydrochinolin,

6-Chloro-2,2-dimethyl-1,2,3,4-tetrahydrochinolin,

8-Chloro-2,2,4-trimethyl-1,2,3,4-tetrahydrochinolin,

8-Bromo-2,2-dimethyl-1,2,3,4-tetrahydrochinolin,

2-Isopropyl-6-methyl-1,2,3,4-tetrahydrochinolin,

2,2,6-Trimethyl-1,2,3,4-tetrahydrochinolin,

2,2,8-Trimethyl-1,2,3,4-tetrahydrochinolin,

2,2-Dimethyl-8-methoxy-1,2,3,4-tetrahydrochinolin,

8-Methoxy-2,2,4-trimethyl-1,2,3,4-tetrahydrochinolin,

8-Isopropoxy-2,2,-dimethyl-1,2,3,4-tetrahydrochinolin,

2,2-Dimethyl-8-methylthio-1,2,3,4-tetrahydrochinolin,

2,2-Dimethyl-8-phenoxy-1,2,3,4-tetrahydrochinolin,

2-Methyl-8-phenylthio-1,2,3,4-tetrahydrochinolin,

2-Methyl-8-nitro-1,2,3,4-tetrahydrochinolin,

2-Methyl-8-trifluoromethyl-1,2,3,4-tetrahydrochinolin,

2,2-Dimethyl-8-trifluoromethyl-1,2,3,4-tetrahydrochinolin,

8-Cyano-2,2-dimethyl-1,2,3,4-tetrahydrochinolin,

6,8-Dichloro-2,2,4-trimethyl-1,2,3,4-tetrahydrochinolin,

7,8-Dichloro-2,2,-dimethyl-1,2,3,4-tetrahydrochinolin,

7,8-Dichloro-2,2,4-trimethyl-1,2,3,4-tetrahydrochinolin,

2,2,6,8-Tetramethyl-1,2,3,4-tetrahydrochinolin,

7-Chloro-2,8-dimethyl-1,2,3,4-tetrahydrochinolin,

7-Chloro-2-ethyl-8-methyl-1,2,3,4-tetrahydrochinolin,

8-Chloro-2,2-dimethyl-1,2,3,4-tetrahydrochinolin,

7-Chloro-2,2,8-trimethyl-1,2,3,4-tetrahydrochinolin,

8-Chloro-2,2,7-trimethyl-1,2,3,4-tetrahydrochinolin,

2,2,-Dimethyl-6-fluoro-1,2,3,4-tetrahydrochinolin,

2,2-Dimethyl-6-methoxy-1,2,3,4-tetrahydrochinolin,

2,2-Dimethyl-6-nitro-1,2,3,4-tetrahydrochinolin,

8-Ethyl-2-methyl-1,2,3,4-tetrahydrochinolin,

2,8-Dimethyl-7-fluoro-1,2,3,4-tetrahydrochinolin,

2,2,-Dimethyl-1,2-dihydrochinolin,

2,2,4-Trimethyl-1,2-dihydrochinolin,

6-Chloro-2,2-dimethyl-1,2,-dihydrochinolin,

2,2,-Dimethyl-6-fluoro-1,2-dihydrochinolin,

2,2,6-Trimethyl-1,2-dihydrochinolin,

6-Chloro-2,2,4-trimethyl-1,2-dihydrochinolin,

4-Chloro-2,2-dimethyl-1,2-dihydrochinolin,

4,6-Dichloro-2,2-dimethyl-1,2-dihydrochinolin,

4-Chloro-2,2,6-trimethyl,1,2-dihydrochinolin,

6,8-Dichloro-4-methyl-1,2-dihydrochinolin,

6,8-Dichloro-2,2-dimethyl-1,2-dihydrochinolin,

2,2-Dimethyl-6-methoxy-1,2,-dihydrochinolin,

2,2-Dimethyl-6-trifluoromethyl-1,2-dihydrochinolin,

2,2,6,8-Tetramethyl-1,2-dihydrochinolin,

7,8-Dichloro-2,2-dimethyl-1,2-dihydrochinolin,

7-Chloro-2,2,8-trimethyl-1,2-dihydrochinolin,

7-Chloro-1,2,3,4,4a,9,9a,10-octahydroacridin,

7-Methyl-1,2,3,4,4a,9,9a,10-octahydroacridin,

7-Methoxy-1,2,3,4,4a,9,9a,10-octahydroacridin,

5-Trifluoromethyl-1,2,3,4,4a,9,9a,10-octahydroacridin,

7-Trifluoromethyl-1,2,3,4,4a,9,9a,10-octahydroacridin,

6-Chloro-5-methyl-1,2,3,4,4a,9,9a,10-octahydroacridin,

5,6-Dichloro-1,2,3,4,4a,9,9a,10-octahydroacridin,

5,7-Dichloro-1,2,3,4,4a,9,9a,10-octahydroacridin,

4-Chloro-2,2,7,8-tetramethyl-1,2-dihydrochinolin,

2,2-Dimethyl-4,7,8-trichloro-1,2-dihydrochinolin,

4,6-Dichloro-2,2-dimethyl-1,2-dihydrochinolin,

2,3,7,8-Tetramethyl-1,2,3,4-tetrahydrochinolin,

7,8-Dimethyl-2-ethyl-1,2,3,4-tetrahydrochinolin,

7,8-Dimethyl-2-isopropyl-1,2,3,4-tetrahydrochinolin,

8-Chloro-2,2-dimethyl-1,2-dihydrochinolin,

6-Chloro-2,2,8-trimethyl-1,2-dihydrochinolin,

6-Ethoxy-2,2-dimethyl-1,2-dihydrochinolin,

6-Ethoxy-2,2,4-trimethyl-1,2-dihydrochinolin,

6,8-Dichloro-2,2-dimethyl-1,2,3,4-tetrahydrochinolin,

6-Chloro-2,2,8-trimethyl-1,2,3,4-tetrahydrochinolin,

6-Ethoxy-2,2,-dimethyl-1,2,3,4-tetrahydrochinolin und

6-Ethoxy-2,2,4-trimethyl-1,2,3,4-tetrahydrochinolin.

Von den Verbindungen der allgemeinen Formel (II) können die 1,2,3,4-Tetrahydrochinoline in einfacher Weise durch Reduktion der entsprechenden Chinoline mittels üblicher Arbeitsweisen erhalten werden, wie sie beispielsweise in US-PS 2 786 845 und US-PS 2 707 681 offenbart sind. Praktisch sämtliche der entsprechenden substituierten Chinoline, die zur Gewinnung der 1,2,3,4-Tetrahydrochinoline eingesetzt werden, können entweder mit Hilfe der Skraup-Reaktion (vgl. Org. Reactions, Band 7, S. 59-98, und J. Org. Chem. 8, S. 544) oder der Doebner-Miller-Reaktion (vgl. Can. J. Chem. 56, S. 632) synthetisiert werden. Beispielsweise kann 2,7,8-Trimethylchinolin aus 2,3-Dimethylanilin und Aldehyd synthetisiert werden, und 8-Chloro-3,7-dimethylchinolin kann aus 2-Chloro-3-methylanilin und α-Methylacrolein synthetisiert werden.

Auf diese Weise kann durch Kombination eines geeigneten Kondensationsmittels mit einem substituierten Anilin ein Chinolin mit Substituenten hergestellt werden, und durch Reduzieren des erhaltenen Produkts läßt sich das entsprechende 1,2,3,4-Tetrahydrochinolin gewinnen.

Von den Verbindungen der allgemeinen Formel (II) können die 1,2-Dihydrochinoline durch Cyclisieren der entsprechenden N-2-Propinyl-substituierten Aniline (J. Medicinal Chem. 16, S. 251) erhalten werden.

Andererseits lassen sich die 2,2,4-Trimethyl-1,2-dihydrochinoline beispielsweise durch Kondensieren eines substitutierten Anilins mit der doppelten Stoffmenge Aceton erhalten (J. Medicinal Chem. 16, S. 251, und J. Org. Chem. 26, S. 1287).

Weiterhin können die 1,2-Dihydrochinoline auch mit Hilfe der Doebner-Miller-Reakton (Can. J. Chem. 56, S. 632) synthetisiert werden. Die 4-Chloro-1,2-dihydrochinoline können auch mit Hilfe des in der oben genannten Literaturstelle J. Medicinal Chem. 16 , S. 251, offenbarten Verfahrens hergestellt werden. Die 1,2,3,4-Tetrahydrochinoline können auch durch Reduktion dieser 1,2-Dihydrochinoline gewonnen werden.

Die 1,2,3,4,4a,9,9a,10-Octahydroacridine können durch Reduzieren der entsprechenden 1,2,3,4-Tetrahydroacridine synthetisiert werden [J. Chem. Soc. 1963 , S. 330].

In dem Verfahren (a) ist N,N'-Carbonyldiimidazol der Formel (III) eine bekannte Verbindung, die in Beilstein, Band 23, II, Seite 35 offenbart ist.

In dem Verfahren (b) bezeichnet die Formel -(IV) Verbindungen, in denen X, n, $Y^1$, $Y^2$, $Y^3$, $Y^4$, $Y^5$ und $Y^6$ die oben angegebenen Bedeutungen haben, und vorzugsweise entsprechen X, n, $Y^1$, $Y^2$, $Y^3$, $Y^4$, $Y^5$, und $Y^6$ den oben als bevorzugt angegebenen Bedeutungen.

Die Verbindungen der Formel (IV) sind neue Verbindungen, über die vor dem Prioritätsdatum der vorliegenden Anmeldung in bekannten Veröffentlichungen noch nicht berichtet worden ist. Als spezielle Beispiele seien die folgenden Verbindungen aufgeführt:

8-Chloro-2-methyl-1(H),2,3,4-tetrahydrochinolin-1-ylcarbonylchlorid,

8-Fluoro-2-methyl-1(H),2,3,4-tetrahydrochinolin-1-ylcarbonylchlorid,

2,8-Dimethyl-1(H),2,3,4-tetrahydrochinolin-1-ylcarbonylchlorid,

8-Ethoxy-2-methyl-1(H),2,3,4-tetrahydrochinolin-1-ylcarbonylchlorid,

2,7,8-Trimethyl-1(H),2,3,4-tetrahydrochinolin-1-ylcarbonylchlorid,

7,8-Dichloro-3-methyl-1(H),2,3,4-tetrahydrochinolin-1-ylcarbonylchlorid,

6,8-Dichloro-4-methyl-1(H),2,3,4-tetrahydrochinolin-1-ylcarbonylchlorid,

7,8-Dichloro-4-methyl-1(H),2,3,4-tetrahydrochinolin,-

1-ylcarbonylchlorid,

2,2,7,8-Tetramethyl-1(H),2,3,4-tetrahydrochinolin-1-ylcarbonylchlorid,

2,4,7,8-Tetramethyl-1(H),2,3,4-tetrahydrochinolin-1-ylcarbonylchlorid,

2,2,4,7,8-Pentamethyl-1(H),2,3,4-tetrahydrochinolin-1-ylcarbonylchlorid,

7,8-Dichloro-1(H),2-dihydrochinolin-1-ylcarbonylchlorid,

2,2,7,8-Tetramethyl-1(H),2-dihydrochinolin-1-ylcarbonylchlorid,

2,2,4,7,8-Pentamethyl-1(H),2-dihydrochinolin-1-ylcarbonylchlorid,

1,2,3,4,4a,9,9a,10(H)-Octahydroacridin-10-ylcarbonylchlorid,

5-Chloro-1,2,3,4,4a,9,9a,10(H)-octahydroacridin-10-ylcarbonylchlorid,

5-Methyl-1,2,3,4,4a,9,9a,10(H)-octahydroacridin-10-ylcarbonylchlorid,

5,6-Dimethyl-1,2,3,4,4a,9,9a,10(H)-octahydroacridin-10-ylcarbonylchlorid,

2-Methyl-1(H),2,3,4-tetrahydrochinolin-1-ylcarbonylclorid,

3-Methyl-1(H),2,3,4-tetrahydrochinolin-1-ylcarbonylchlorid,

4-Methyl-1(H),2,3,4-tetrahydrochinolin-1-ylcarbonylchlorid,

2-Ethyl-1(H),2,3,4-tetrahydrochinolin-1-ylcarbonylchlorid,

2-Propyl-1(H),2,3,4-tetrahydrochinolin-1-ylcarbonylchlorid,

2-Isopropyl-1(H),2,3,4-tetrahydrochinolin-1-ylcarbonylchlorid,

2,2-Dimethyl-1(H),2,3,4-tetrahydrochinolin-1-ylcarbonylchlorid,

2,2-Diethyl-1(H),2,3,4-tetrahydrochinolin-1-ylcarbonylchlorid,

2,2,4-Trimethyl-1(H),2,3,4-tetrahydrochinolin-1-ylcarbonylchlorid,

8-Chloro-2-ethyl-1(H),2,3,4-tetrahydrochinolin-1-ylcarbonylchlorid,

6-Chloro-2,2-dimethyl-1(H),2,3,4-tetrahydrochinolin-1-ylcarbonylchlorid,

8-Chloro-2,2,4-trimethyl-1(H),2,3,4-tetrahydrochinolin-1-ylcarbonylchlorid,

8-Bromo-2,2-dimethyl-1(H),2,3,4-tetrahydrochinolin-1-ylcarbonylchlorid,

2-Isopropyl-6-methyl-1(H),2,3,4-tetrahydrochinolin-1-ylcarbonylchlorid,

2,2,6-Trimethyl-1(H),2,3,4-tetrahydrochinolin-1-ylcarbonylchlorid,

2,2,8-Trimethyl-1(H),2,3,4-tetrahydrochinolin-1-ylcarbonylchlorid,

2,2-Dimethyl-8-methoxy-1(H),2,3,4-tetrahydrochinolin-1-ylcarbonylchlorid,

8-Methoxy-2,2,4-trimethyl-1(H),2,3,4-tetrahydrochinolin-1-ylcarbonylchlorid,

8-Isopropoxy-2,2-dimethyl-1(H),2,3,4-tetrahydrochinolin-1-ylcarbonylchlorid,

2,2-Dimethyl-8-methylthio-1(H),2,3,4-tetrahydrochinolin-1-ylcarbonylchlorid,

2,2-Dimethyl-8-phenoxy-1(H),2,3,4-tetrahydrochinolin-1-ylcarbonylchlorid,

2-Methyl-8-phenylthio-1(H),2,3,4-tetrahydrochinolin-1-ylcarbonylchlorid,

2-Methyl-8-nitro-1(H),2,3,4-tetrahydrochinolin-1-ylcarbonylchlorid,

2-Methyl-8-trifluoromethyl-1(H),2,3,4-tetrahydrochinolin-1-ylcarbonylchlorid,

2,2-Dimethyl-8-trifluoromethyl-1(H),2,3,4-tetrahydrochinolin-1-ylcarbonylchlorid,

8-Cyano-2,2-dimethyl-1(H),2,3,4-tetrahydrochinolin-1-ylcarbonylchlorid,

6,8-Dichloro-2,2,4-trimethyl-1(H),2,3,4-tetrahydrochinolin-1-ylcarbonylchlorid,

7,8-Dichloro-2,2-dimethyl-1(H),2,3,4-tetrahydrochinolin-1-ylcarbonylchlorid,

7,8-Dichloro-2,2,4-trimethyl-1(H),2,3,4-tetrahydrochinolin-1-ylcarbonylchlorid,

2,2,6,8-Tetramethyl-1(H),2,3,4-tetrahydrochinolin-1-ylcarbonylchlorid,

7-Chloro-2,8-dimethyl-1(H),2,3,4-tetrahydrochinolin-1-ylcarbonylchlorid,

7-Chloro-2-ethyl-8-methyl-1(H),2,3,4-tetrahydrochinolin-1-ylcarbonylchlorid,

8-Chloro-2,2-dimethyl-1(H),2,3,4-tetrahydrochinolin-1-ylcarbonylchlorid,

7-Chloro-2,2,8-trimethyl-1(H),2,3,4-tetrahydrochinolin-1-ylcarbonylchlorid,

8-Chloro-2,2,7-trimethyl-1(H),2,3,4-tetrahydrochinolin-1-ylcarbonylchlorid,

2,2-Dimethyl-6-fluoro-1(H),2,3,4-tetrahydrochinolin-1-ylcarbonylchlorid,

2,2-Dimethyl-6-methoxy-1(H),2,3,4-tetrahydrochinolin-1-ylcarbonylchlorid,

2,2-Dimethyl-6-nitro-1(H),2,3,4-tetrahydrochinolin-1-ylcarbonylchlorid,

8-Ethyl-2-methyl-1(H),2,3,4-tetrahydrochinolin-1-ylcarbonylchlorid,

2,8-Dimethyl-7-fluoro-1(H),2,3,4-tetrahydrochinolin-1-ylcarbonylchlorid,

2,2-Dimethyl-1(H),2-dihydrochinolin-1-ylcarbonylchlorid,

2,2,4-Trimethyl-1(H),2-dihydrochinolin-1-ylcarbonylchlorid,

6-Chloro-2,2-dimethyl-1(H),2-dihydrochinolin-1-ylcarbonylchlorid,

2,2-Dimethyl-6-fluoro-1(H),2-dihydrochinolin-1-ylcarbonylchlorid,

2,2,6-Trimethyl-1(H),2-dihydrochinolin-1-ylcarbonylchlorid,

6-Chloro-2,2,4-trimethyl-1(H),2-dihydrochinolin-1-ylcarbonylchlorid,

4-Chloro-2,2-dimethyl-1(H),2-dihydrochinolin-1-ylcarbonylchlorid,

4,6-Dichloro-2,2-dimethyl-1(H),2-dihydrochinolin-1-ylcarbonylchlorid,

4-Chloro-2,2,6-trimethyl-1(H),2-dihydrochinolin-1-ylcarbonylchlorid,

6,8-Dichloro-4-methyl-1(H),2-dihydrochinolin-1-ylcarbonylchlorid,

6,8-Dichloro-2,2-dimethyl-1(H),2-dihydrochinolin-1-ylcarbonylchlorid,

2,2-Dimethyl-6-methoxy-1(H),2-dihydrochinolin-1-ylcarbonylchlorid,

2,2-Dimethyl-6-trifluoromethyl-1(H),2-dihydrochinolin-1-ylcarbonylchlorid,

2,2,6,8-Tetramethyl-1(H),2-dihydrochinolin-1-ylcarbonylchlorid,

7,8-Dichloro-2,2-dimethyl-1(H),2-dihydrochinolin-1-ylcarbonylchlorid,

7-Chloro-2,2,8-trimethyl-1(H),2-dihydrochinolin-1-ylcarbonylchlorid,

7-Chloro-1,2,3,4,4a,9,9a,10(H)-octahydroacridin-10-ylcarbonylchlorid,

7-Methyl-1,2,3,4,4a,9,9a,10(H)-octahydroacridin-10-ylcarbonylchlorid,

7-Methoxy-1,2,3,4,4a,9,9a,10(H)-octahydroacridin-10-ylcarbonylchlorid,

5-Trifluoromethyl-1,2,3,4,4a,9,9a,10(H)-octahydroacridin-10-yl-carbonylchlorid,

7-Trifluoromethyl-1,2,3,4,4a,9,9a,10(H)-octahydroacridin-10-yl-carbonylchlorid,

6-Chloro-5-methyl-1,2,3,4,4a,9,9a,10(H)-octahydroacridin-10-yl-carbonylchlorid,

5,6-Dichloro-1,2,3,4,4a,9,9a,10(H)-octahydroacridin-10-yl-carbonylchlorid,

5,7-Dichloro-1,2,3,4,4a,9,9a,10(H)-octahydroacridin-10-yl-carbonylchlorid,

4-Chloro-2,2,7,8-tetramethyl-1(H),2-dihydrochinolin-1-ylcarbonylchlorid,

2,2-Dimethyl-4,7,8-trichloro-1(H),2-dihydrochinolin-1-ylcarbonylchlorid,

4,6-Dichloro-2,2-dimethyl-1(H),2-dihydrochinolin-1-ylcarbonylchlorid,

2,3,7,8-Tetramethyl-1(H),2,3,4-tetrahydrochinolin-1-ylcarbonylchlorid,

7,8-Dimethyl-2-ethyl-1(H),2,3,4-tetrahydrochinolin-1-ylcarbonylchlorid,

7,8-Dimethyl-2-isopropyl-1(H),2,3,4-tetrahydrochinolin-1-ylcarbonylchlorid,

8-Chloro-2,2-dimethyl-1(H),2-dihydrochinolin-1-ylcarbonylchlorid,

6-Chloro-2,2,8-trimethyl-1(H),2-dihydrochinolin-1-ylcarbonylchlorid,

6-Ethoxy-2,2-dimethyl-1(H),2-dihydrochinolin-1-ylcarbonylchlorid,

6-Ethoxy-2,2,4-trimethyl-1(H),2-dihydrochinolin-1-ylcarbonylchlorid,

6,8-Dichloro-2,2-dimethyl-1(H),2,3,4-tetrahydrochinolin-1-ylcarbonylchlorid,

6-Chloro-2,2,8-trimethyl-1(H),2,3,4-tetrahydrochinolin-1-ylcarbonylchlorid,

6-Ethoxy-2,2-dimethyl-1(H),2,3,4-tetrahydrochinolin-1-ylcarbonylchlorid und

6-Ethoxy-2,2,4-trimethyl-1(H),2,3,4-tetrahydrochinolin-1-ylcarbonylchlorid.

Die Verbindungen der Formel (IV) werden erhalten mittels eines Verfahrens, in dem

(c) Verbindungen der Formel (II)

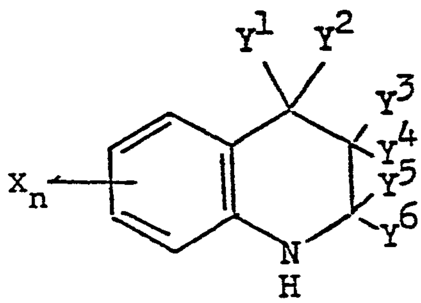

(II)

in der X, $Y^1$, $Y^2$, $Y^3$, $Y^4$, $Y^5$, $Y^6$ und n die oben angegebenen Bedeutungen haben, in bekannter Weise mit Phosgen oder Trichloromethylchlorameisensäureester umgesetzt werden.

Wenn beispielsweise 2,7,8-Trimethyl-methyl-1,2,3,4-tetrahydrochinolin und Trichloromethylchlorameisensäureester als Ausgangsstoffe eingesetzt werden, läßt sich der Ablauf der Reaktion in dem Verfahren (c) durch die folgende Gleichung darstellen:

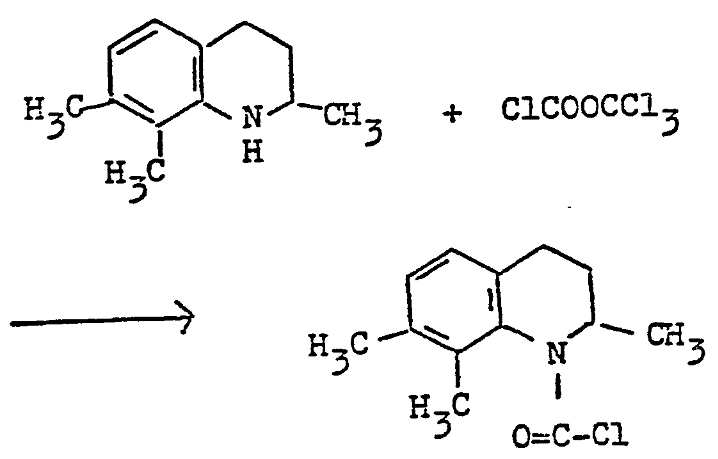

Die Verbindungen der Formel (II) in dem Verfahren (c) sind die gleichen wie diejenigen, die in dem oben beschriebenen Verfahren (a) eingesetzt werden.

Bei der Durchführung des Verfahrens (c) können die gleichen inerten organischen Lösungsmittel verwendet werden, wie sie im Folgenden für das Verfahren (a) aufgeführt sind. Wiederum kann die Reaktion beispielsweise bei einer Temperatur im Bereich zwischen etwa 0°C und dem Siedepunkt der Reaktionsmischung und vorzugsweise unter normalem Atmosphärendruck durchgeführt werden.

Bei der praktischen Durchführung des vorstehenden Verfahrens können die gewünschten Verbindungen der Formel (IV) dadurch hergestellt werden, daß beispielsweise 1 mol der Verbindung der allgemeinen Formel (II) mit 1 bis etwa 10 mol, vorzugsweise etwa 2 bis etwa 5 mol, Phosgen oder mit 1/2 mol bis etwa 10 mol, vorzugsweise 1 mol bis etwa 5 mol, Trichloromethylchlorameisensäureester in einem inerten Lösungsmittel umgesetzt wird, wobei die Temperatur etwa auf Rückflußtemperatur gesteigert wird.

Als geeignete Verdünnungsmittel bei der Durchführung des vorgenannten Verfahrens (a) lassen sich sämtliche inerten organischen Lösungsmittel erwähnen.

Beispiele für solche Lösungsmittel oder Verdünnungsmittel umfassen aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Das Verfahren (a) kann innerhalb eines weiten Temperatur-Bereichs durchgeführt werden.

Beispielsweise kann die Temperatur im Bereich von etwa -20°C und dem Siedepunkt der Mischung liegen, wobei ein Bereich von etwa 0°C bis etwa 100°C bevorzugt wird. Zweckmäßigerweise wird die Reaktion unter normalem atmosphärischen Druck durchgeführt, jedoch ist es möglich, das Verfahren unter erhöhtem oder vermindertem Druck durchzuführen.

Bei der Durchführung des Verfahrens (a) kann die gewünschte neue Verbindung der Formel (I) dadurch erhalten werden, daß in einem inerten Lösungsmittel etwa 1 bis etwa 2 mol, vorzugsweise 1 bis etwa 1,2 mol, N,N'-Carbonyldiimidazol der Formel (III) mit 1 mol der Verbindung der Formel (II) umgesetzt wird.

Bei der praktischen Durchführung des Verfahrens (b) werden zweckmäßigerweise als Verdünnungsmittel die gleichen inerten organischen Lösungsmittel verwendet, wie sie oben beispielhaft für das Verfahren (a) genannt sind. Das Verfahren (b) kann weiterhin in Gegenwart eines säurebindenden Mittels durchgeführt werden. Als säurebindende Mittel erwähnt seien die gewöhnlich verwendeten Hydroxide, Carbonate, Hydrogencarbonate und Alkoholate von Alkalimetallen sowie tertiäre Amine wie Triethylamin, Diethylanilin und Pyridin.

Das Verfahren (b) kann in einem weiten Temperaturbereich durchgeführt werden. Beispielsweise kann es bei einer Temperatur im Bereich von etwa 0°C und dem Siedepunkt der Reaktionsmischung, vorzugsweise von etwa 50°C bis etwa 100°C, durchgeführt werden.

Weiterhin wird die Reaktion vorzugsweise unter normalem Atmosphärendruck durchgeführt, jedoch kann sie auch unter erhöhtem oder vermindertem Druck durchgeführt werden.

Bei der Durchführung des Verfahrens (b) kann die gewünschte neue Verbindung der Formel (I) dadurch erhalten werden, daß z.B. 1 mol der Verbindung der allgemeinen Formel (IV) mit etwa 1 mol bis etwa 5 mol, vorzugsweise etwa 1,1 bis etwa 3 mol Imidazol der Formel (V) in einem inerten Lösungsmittel umgesetzt wird.

Die Verbindungen der Formel (I) gemäß der vorliegenden Erfindung zeigen starke herbizide Aktivität. Demgemäß können diese Verbindungen zur Unkrautbekämpfung verwendet werden. Bei Verwendung als Mittel zur Bodenbehandlung vor dem Auflaufen oder als Mittel zur Behandlung von Laub und Boden zeigen sich auffällige selektive Bekämpfungs-Aktivitäten.

Die Verbindungen der Formel (I) der vorliegenden Erfindung zeichnen sich nicht nur in bezug auf ihre Sicherheit aus, sondern sie haben außerdem eine überragende herbizide Wirkung mit einem breiten herbiziden Spektrum.

Beispielsweise sind die Verbindungen dadurch gekennzeichnet, daß sie ihre Bekämpfungswirkung gegen die nachstehend aufgeführten Reisfeld-Unkräuter entfalten, während sie den Reispflanzen keinerlei Schaden zufügen.

| Pflanzenname: | Lateinische wissenschaftliche Bezeichnung: |
|---|---|
| Dikotyledonen | |
| "Kikashigusa" | Rotala indica Koehne |
| Falsche Pimpernelle (Büchsenkraut) | Lindernia procumbens Philcox |
| Falscher Weiderich (Heusenkraut) | Ludwigia prostrata Roxburgh |
| Breitblättriges Laichkraut | Potamogeton distinctus A. Benn. |
| Amerikanische Wasserwurz (Kreuztännel) | Elatine triandra Schk. |
| Monokotyledonen | |
| Hühnerhirse | Echinochloa crus-galli P. Beauv. var. |
| Monochoria | Monochoria vaginalis Presl |
| Nadel-Sumpfbinse | Eleocharis acicularis L. |
| Wassernuß | Eleocharis kuroguwai Ohwi |
| Schirmkraut (Cypergras) | Cyperus difformis L. |
| Wasser-Erdmandel (Cypergras) | Cyperus serotinus Rottboell |
| "Urikawa" (Pfeilkraut) | Sagittaria pygmaea Miq. |
| Schmalblättriger Wasserwegerich (Froschlöffel) | Alisma canaliculatum A. Br. et Bouchê |
| Simse | Scirpus juncoides Roxburgh var. |

Weiterhin zeigen die Verbindungen der vorliegenden Erfindung herbizide Aktivität gegen die folgenden, auf höher gelegenen Anbauflächen wachsenden Unkräuter.

| Pflanzenname: | Lateinische wissenschaftliche Bezeichnung: |
|---|---|
| **Dikotyledonen** | |
| Knöterich | Polygonum sp. |
| Gänsefuß | Chenopodium album Linnaeus |
| Vogelmiere | Stellaria media Villars |
| Kohl-Portulak | Portulaca oleracea Linnaeus |
| **Monokotyledonen** | |
| Hühnerhirse | Echinochloa crus-galli P.Beauv. |
| Fingergras | Digitaria adscendens Henr. |
| Erdmandel (Riedgras) | Cyperus iria L. |

Die im Vorstehenden angegebenen Pflanzenklassen sind jedoch nur typische Beispiele für die jeweils mit der lateinischen wissenschaftlichen Bezeichnung genannte Gattung.

Die Einsetzbarkeit der Wirkstoffe gemäß der vorliegenden Erfindung ist jedoch in keiner Weise auf diese Gattungen beschränkt sondern erstreckt sich gleicher Weise auch auf andere Pflanzen.

In Abhängigkeit von den Konzentrationen eignen sich die Verbindungen zur totalen Bekämpfung von Unkräutern, beispielsweise auf Industriegelände und Bahnkörpern sowie auf Wegen und Plätzen mit oder ohne Baumbestand. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe der Formel (I) zeigen außerdem eine ausgezeichnete fungizide Aktivität und können zur Bekämpfung unerwünschter Pflanzenpathogene eingesetzt werden.

Wenn die Wirkstoffe der vorliegenden Erfindung als Fungizide für Landwirtschaft und Gartenbau eingesetzt werden, sind sie wirksam gegen verschiedene Pflanzenkrankheiten, die beispielsweise durch Plasmodiophoromycetes, Oomycetes, Chytridiomycetes, Zygomycetes, Ascomycetes, Basidiomycetes und Deuteromycetes verursacht werden.

Zu den Pflanzenkrankheiten, gegen die die Verbindungen der vorliegenden Erfindung mit Wirkamkeit eingesetzt werden, zählen beispielsweise Pyricularia oryzae bei Reis, Ophiobolus miyabebnus bei Reis, Alternaria brassicae bei Kreuzblütlern, Alternaria mali bei Äpfeln, Alternaria kukuchiana bei Birnen, Phytophthora infestans bei verschiedenen Nutzpflanzen, Colletotrichum lagenarium bei verschiedenen Gurken-und Melonen-Species sowie Botrytis cinerea bei verschiedenen Nutzpflanzen. Weiterhin zu erwähnen ist Xanthomonas oryzae, eine wichtige bakterielle Erkrankung.

Die gute Tolerierung der Wirkstoffe durch die Pflanzen bei den zur Bekämpfung der Pflanzenerkrankungen erforderlichen Konzentrationen erlaubt die Behandlung der oberirdischen Pflanzenteile, der Teile der vegetativen Fortpflanzung und der Samen sowie des Bodens.

Die Wirkstoffe können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut -(Beizmittel) sowie Formulierungen für den Einsatz mit Verbrennungseinrichtungen wei Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der Wirkstoffe mit Streckmitteln, das heißt mit flüssigen oder verflüssigten gasförmigen oder festen Verdünnungsmitteln oder Trägern, gegebenenfalls unter Verwendung

grenzflächenaktiver Mittel, das heißt von Emulgatoren und/oder Dispergiermitteln und/oder - schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe und ebenso auch Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid, und Preußisch Blau und organische Farbstoffe wie Alizarin-Farbstoffe, Azo-Farbstoffe oder metallphthalocyanin-Farbstoffe, swoie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der vorliegenden Erfindung können in ihren oben beschriebenen Formulierungen oder ihren verschiedenen Anwendungsformen gemeinsam mit anderen aktiven Verbindungen vorliegen, etwa mit Germiziden (Fungiziden und Bakteriziden), Insektiziden, Akariziden, Nematiziden, Herbiziden, vogelabweisenden Mitteln, Wachstumsregulatoren, Düngemitteln und/oder Mitteln zur Bodenverbesserung. Solche Mischungen können zu gebrauchsfertigen Präparaten formuliert werden oder im Tank vermischt werden.

Die aktiven Verbindungen gemäß der vorliegenden Erfindung können direkt als solche eingesetzt werden, oder sie können in Form solcher Präparate wie Präparaten zum Flüssigversprühen, Emulsionen, Suspensionen, als Stäubemittel, Paste oder Granulat angewandt werden. Oder aber sie können nach wieterer Verdünnung verwendet werden.

Wenn die aktive Verbindung als Herbizid eingesetzt werden soll, kann sie mit Hilfe solcher Arbeitsgänge wie Bewässern, Spritzen, Zerstäuben oder Streuen von Granulat aufgebracht werden. Wenn sie andererseits als Fungizid in der Landwirtschaft bzw. im Gartenbau eingesetzt werden soll, kann sie mittels solcher Arbeitsgänge wie Bewässern, Spritzen, Zerstäuben, Vernebeln, Verdampfen, Gießen, Suspensionsbildung, Beschichten, Stäuben, Staubdüngung, Naßdüngung, Naßbedecken, Pastenbedecken, und "Gewandbedecken" ("robe covering") zur Anwendung gebracht werden.

Beim Einsatz als Herbizid können die Wirkstoffe gemäß der vorliegenden Erfindung entweder vor oder nach der Keimung der Pflanzen angewandt werden. Es ist auch möglich, sie vor der Aussaat in den Boden einzuarbeiten.

Die Konzentration der Wirkstoffe kann innerhalb eines breiten Bereichs variieren. Dieser hängt grundsätzlich von den gewünschten Ergebnissen ab. Wenn die Verbindung als Herbizid eingesetzt werden soll, wird sie beispielsweise in einer Konzentration von etwa 0,05 bis etwa 5 kg/ha, vorzugsweise von etwa 0,2 bis etwa 3 kg/ha, angewandt.

Wenn die Verbindung andererseits als land-und gartenwirtschaftliches Fungizid eingesetzt werden soll, wird sie in einer Konzentration von beispielsweise etwa 0,0001 bis etwa 1 Gew.-%, und vorzugsweise von etwa 0,001 bis etwa 0,5 Gew.-%, angewandt.

Im Fall der Saatgut-Behandlung kann die aktive Verbindung in einer Menge von beispielsweise etwa 0,001 bis etwa 50 g, vozugsweise etwa 0,01 bis 10 g auf 1 kg des Saatguts zu Anwendung gebracht werden.

Wo der Boden behandelt werden soll, kann die aktive Verbindung auf den gewünschten Wirkungsort beispielsweise in einer Konzentration von etwa 0,00001 bis etwa 0,1 Gew.-%, und insbesondere von etwa 0,00001 bis etwa 0,2 Gew.-%, aufgebracht werden.

Die folgenden Beispiele erläutern die vorliegende Erfindung im einzelnen. Die Erfindung ist jedoch nicht auf diese Beispiele beschränkt.

Herstellungsbeispiele

Beispiel1

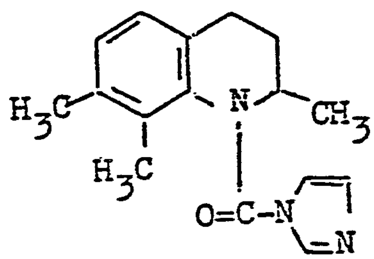

(Verbindung Nr. 1)

2,7,8-Trimethyl-1(H),2,3,4-tetrahydrochinolin-1-ylcarbonylchlorid (2,4 g) und Imidazol (20 g) wurden in 20 ml Tetrahydrofuran 16 h bei Rückfluß-Temperatur gerührt. Das Tetrahydrofuran und das überschüssige Imidazol wurden unter vermindertem Druck abdestilliert. Der Rückstand wurde durch Zusatz von Wasser kristallisiert. Die erhaltenene Kristalle wurden abfiltriert, wonach 2,2 g des gewünschten 2,7,8-Trimethyl-1(H),2,3,4-tetrahydrochinolin-1-carbonylimidazols erhalten wurden. Dieses wurde dann aus Diethylether umkristallisiert. Schmp. der Verbindung 86-88°C.

Beispiel 2

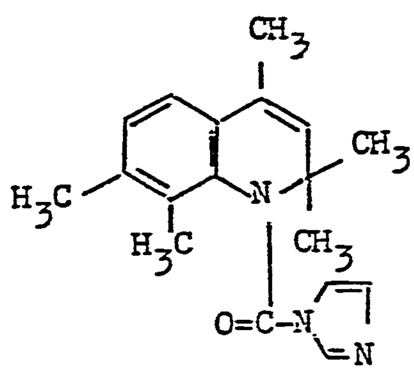

(Verbindung Nr. 18)

2,2,4,7,8-Pentamethyl-1,2-dihydrochinolin (2,0 g) und N,N'-Carbonyldiimidazol (1,6 g) wurden in 20 ml Toluol 24 h unter Rühren erhitzt. Nach dem Abdestillieren des Lösungsmittels unter vermindertem Druck wurde der Rückstand einem Vakuum von 1,33 mbar (1 mmHg) bei 80°C ausgesetzt. Der Rückstand wurde dann durch Zusatz von Wasser kristallisiert. Die erhaltenen Kristalle wurden abfiltriert, wonach 2,1 g des gewünschten 2,2,4,7,8-Pentamethyl-1,2-dihydrochinolin-1-ylcarbonylimidazols erhalten wurden. Dieses wurde dann aus Diethylether umkristallisiert. Schmp. der Verbindung 88-91°C.

Verbindungen der allgemeinen Formel (I) gemäß der vorliegenden Erfindung, die nach den gleichen Arbeitsweisen, wie sie in den Beispielen 1 und 2 beschrieben sind, synthetisiert wurden, sind in der nachstehenden Tabelle 1 aufgeführt.

Tabelle 1

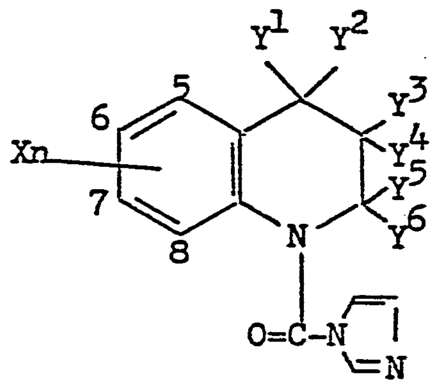

| Verbindung Nr. | 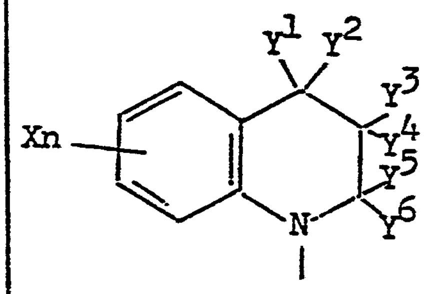 | Physikalische Konstante Schmelzpunkt (Schmp.) |
|---|---|---|
| 2 | 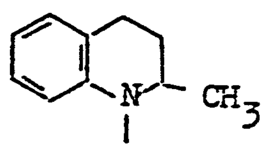 | Schmp. 81-84°C |
| 3 | 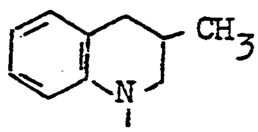 | Schmp. 80-82.5°C |
| 4 | 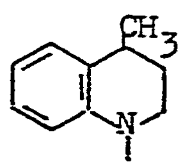 | Schmp. 95 - 97°C |
| 5 | 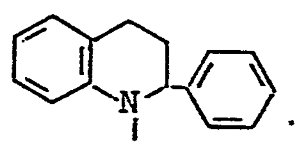 | Schmp. 125-127°C |
| 6 | 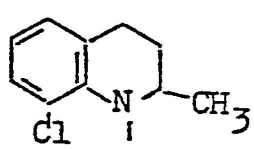 | Schmp. 138-140°C |
| 7 | 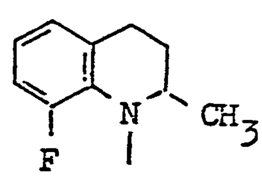 | Schmp. 95-98°C |

Tabelle 1 - Fortsetzung

| Verbindung Nr. | 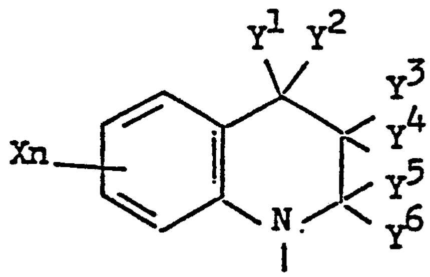 | Physikalische Konstante Schmelzpunkt (Schmp.) |
|---|---|---|
| 8 | 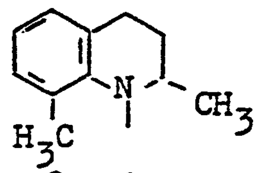 | Schmp. 97-100°C |
| 9 | 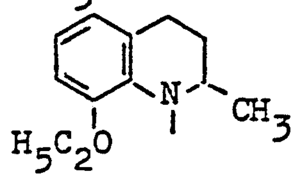 | Schmp. 70-75°C |
| 10 | 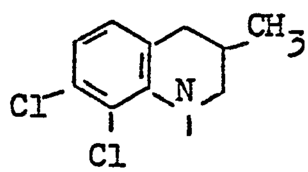 | Schmp. 111-114°C |
| 11 | 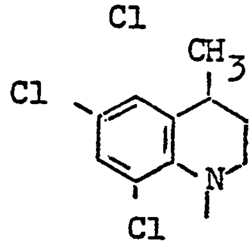 | Schmp. 126-128°C |
| 12 | 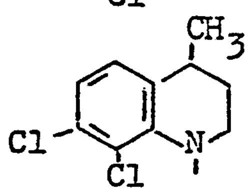 | Schmp. 110-113°C |
| 13 | 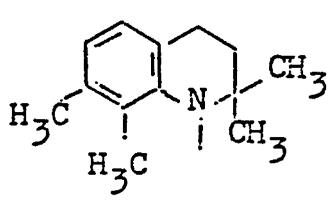 | |

Tabelle 1 - Fortsetzung

| Verbindung Nr. | 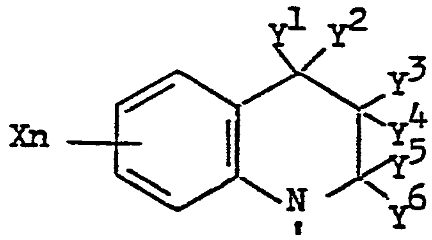 | Physikalische Konstante Schmelzpunkt (Schmp.) |
| --- | --- | --- |
| 14 | 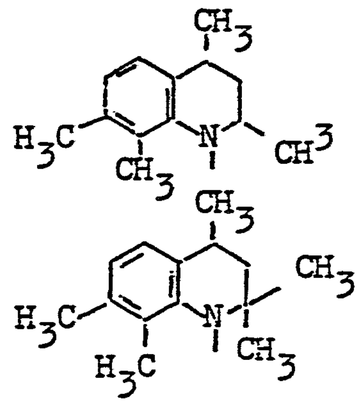 | |
| 15 | | |
| 16 | 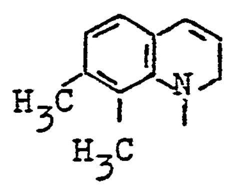 | |
| 17 | 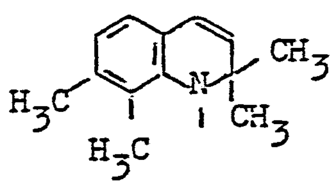 | |
| 19 | 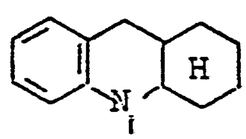 | Schmp. 130-132°C |
| 20 | 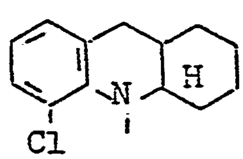 | Schmp. 127-129°C |

Tabelle 1 - Fortsetzung

| Verbindung Nr. | 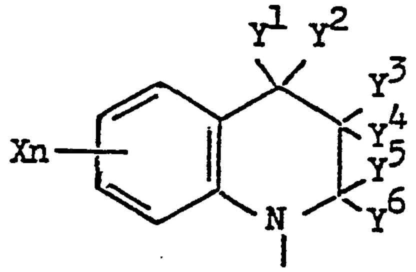 | Physikalische Konstante Schmelzpunkt (Schmp.) |
|---|---|---|
| 21 | 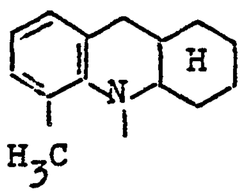 | Schmp. 127-129°C |
| 22 | 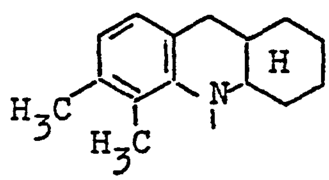 | Schmp. 121-124°C |

Zusätzlich zu den im Vorstehenden beispielhaft genannten Verbindungen werden andere spezielle Beispiele für die Verbindungen der allgemeinen Formel (I), die nach ähnlichen Arbeitsweisen wie in den vorstehenden Beispielen erhalten werden, im Folgenden aufgeführt.

Nr. 23 2-Ethyl-1 (H),2,3,4-tetrahydrochinolin-1-ylcarbonylimidazol,

Nr. 24 2-Propyl-1 (H),2,3,4,-tetrahydrochinolin-1-ylcarbonylimidazol,

Nr. 25 2-Isopropyl-1 (H),2,3,4-tetrahydrochinolin-1-ylcarbonylimidazol,

Nr. 26 2,2-Dimethyl-1 (H),2,3,4-tetrahydrochinolin-1-ylcarbonylimidazol, $n_D^{20}$ 1,57 34,

Nr. 27 2,2-Diethyl-1 (H),2,3,4-tetrahydrochinolin-1-ylcarbonylimidazol,

Nr. 28 2,2,4-Trimethyl-1 (H),2,3,4-tetrahydrochinolin-1-ylcarbonylimidazol, $n_D^{20}$ 1,5708,

Nr. 29 8-Chloro-2-ethyl-1 (H),2,3,4-tetrahydrochinolin-1-ylcarbonylimidazol,

Nr. 30 6-Chloro-2,2-dimethyl-1 (H),2,3,4-tetrahydrochinolin-1-ylcarbonylimidazol,

Nr. 31 8-Chloro-2,2,4-trimethyl-1 (H),2,3,4-tetrahydrochinolin-1-ylcarbonylimidazol,

Nr. 32 8-Bromo-2,2-dimethyl-1 (H),2,3,4-tetrahydrochinolin-1-ylcarbonylimidazol,

Nr. 33 2-Isopropyl-6-methyl-1 (H),2,3,4-tetrahydrochinolin-1-ylcarbonylimidazol,

Nr. 34 2,2,6-Trimethyl-1 (H),2,3,4-tetrahydrochinolin-1-ylcarbonylimidazol, Schmp. 122-125°C,

Nr. 35 2,2,8-Trimethyl-1 (H),2,3,4-tetrahydrochinolin-1-ylcarbonylimidazol,

Nr. 36 2,2-Dimethyl-8-methoxy-1 (H),2,3,4-tetrahydrochinolin-1-ylcarbonylimidazol,

Nr. 37 8-Methoxy-2,2-4-trimethyl-1 (H),2,3,4-tetrahydrochinolin-1-ylcarbonylimidazol,

Nr. 38 2,2-Dimethyl-8-isopropoxy-1 (H),2,3,4-tetrahydrochinolin-1-ylcarbonylimidazol,

Nr. 39 2,2-Dimethyl-8-methylthio-1 (H),2,3,4-tetrahydrochinolin-1-ylcarbonylimidazol,

Nr. 40 2,2-Dimethyl-8-phenoxy-1 (H),2,3,4-tetrahydrochinolin-1-ylcarbonylimidazol,

Nr. 41 2-Methyl-8-phenylthio-1 (H),2,3,4-tetrahydrochinolin-1-ylcarbonylimidazol,

Nr. 42 2-Methyl-8-nitro-1 (H),2,3,4-tetrahydrochinolin-1-ylcarbonylimidazol,

Nr. 43 2-Methyl-8-trifluoromethyl-1 (H),2,3,4-tetrahydrochinolin-1-ylcarbonylimidazol,

Nr. 44 2,2-Dimethyl-8-trifluoromethyl-1 (H)-,2,3,4-tetrahydrochinolin-1-ylcarbonylimidazol,

Nr. 45 8-Cyano-2,2-dimethyl-1 (H),2,3,4-tetrahydrochinolin-1-ylcarbonylimidazol,

Nr. 46 6,8-Dichloro-2,2,4-trimethyl-1 (H),2,3,4-tetrahydrochinolin-1-ylcarbonylimidazol,

Nr. 47 7,8-Dichloro-2,2-dimethyl-1 (H),2,3,4-

tetrahydrochinolin-1-ylcarbonylimidazol,
Nr. 48 7,8-Dichloro-2,2,4-trimethyl-1 (H),2,3,4-tetrahydrochinolin-1-ylcarbonylimidazol,
Nr. 49 2,2,6,8-Tetramethyl-1 (H),2,3,4-tetrahydrochinolin-1-ylcarbonylimidazol,
Nr. 50 7-Chloro-2,8-dimethyl-1 (H),2,3,4-tetrahydrochinolin-1-ylcarbonylimidazol,
Nr. 51 7-Chloro-2-ethyl-8-methyl-1 (H),2,3,4-tetrahydrochinolin-1-ylcarbonylimidazol,
Nr. 52 8-Chloro-2,7-dimethyl-1 (H),2,3,4-tetrahydrochinolin-1-ylcarbonylimidazol,
Nr. 53 7-Chloro-2,2,8-trimethyl-1 (H),2,3,4-tetrahydrochinolin-1-ylcarbonylimidazol,
$n_D^{20}$ 1,5687,
Nr. 54 8-Chloro-2,2,7-trimethyl-1 (H),2,3,4-tetrahydrochinolin-1-ylcarbonylimidazol,
Nr. 55 2,2-Dimethyl-6-fluoro-1 (H),2,3,4-tetrahydrochinolin-1-ylcarbonylimidazol,
Nr. 56 2,2-Dimethyl-6-methoxy-1 (H),2,3,4-tetrahydrochinolin-1-ylcarbonylimidazol,
Nr. 57 2,2-Dimethyl-6-nitro-1 (H),2,3,4-tetrahydrochinolin-1-ylcarbonylimidazol,
Nr. 58 8-Ethoxy-2-methyl-1 (H),2,3,4-tetrahydrochinolin-1-ylcarbonylimidazol,
Nr. 59 2,8-Dimethyl-7-fluoro-1 (H),2,3,4-tetrahydrochinolin-1-ylcarbonylimidazol,
Nr. 60 2,2-Dimethyl-1 (H),2-dihydrochinolin-1-ylcarbonylimidazol,
Nr. 61 2,2,4-Trimethyl-1 (H),2-dihydrochinolin-1-ylcarbonylimidazol,
Nr. 62 6-Chloro-2,2-dimethyl-1 (H),2-dihydrochinolin1-ylcarbonylimidazol,
$n_D^{30}$ 1,6004,
Nr. 63 2,2-Dimethyl-6-fluoro-1 (H),2-dihydrochinolin-1-ylcarbonylimidazol,
Nr. 64 2,2,6-Trimethyl-1 (H),2-dihydrochinolin-1-ylcarbonylimidazol, $n_D^{20}$ 1,5908,
Nr. 65 6-Chloro-2,2,4-trimethyl-1 (H),2-dihydrochinolin-1-ylcarbonylimidazol,
Nr. 66 4-Chloro-2,2-dimethyl-1 (H),2-dihydrochinolin-1-ylcarbonylimidazol,
Nr. 67 4,6-Dichloro-2,2-dimethyl-1 (H),2-dihydrochinolin-1-ylcarbonylimidazol,
Nr. 68 4-Chloro-2,2,6-trimethyl-1 (H),2-dihydrochinolin-1-ylcarbonylimidazol,
Nr. 69 6,8-Dichloro-4-methyl-1 (H),2-dihydrochinolin-1-ylcarbonylimidazol,
Nr. 70 6,8-Dichloro-2,2-dimethyl-1 (H),2-dihydrochinolin-1-ylcarbonylimidazol,
Nr. 71 2,2-Dimethyl-6-methoxy-1 (H),2-dihydrochinolin-1-ylcarbonylimidazol,
Nr. 72 2,2-Dimethyl-6-trifluoromethyl-1 (H),2-dihydrochinolin-1-ylcarbonylimidazol,
Nr. 73 2,2,6,8-Tetramethyl-1(H),2-dihydrochinolin-1-ylcarbonylimidazol,
Nr. 74 7,8-Dichloro-2,2-dimethyl-1 (H),2-dihydrochinolin-1-ylcarbonylimidazol,
Nr. 75 7-Chloro-2,2,8-trimethyl-1 (H),2-dihydrochinolin-1-ylcarbonylimidazol,
Nr. 76 7-Chloro-1,2,3,4,4a,9,9a,10 (H)-octahydroacridin-10-ylcarbonylimidazol,
Nr. 77 7-Methyl-1,2,3,4,4a,9,9a,10 (H)-octahydroacridin-10-ylcarbonylimidazol,
Nr. 78 7-Methoxy-1,2,3,4,4a,9,9a,10 (H)-octahydroacridin-10-ylcarbonylimidazol,
Nr. 79 5-Trifluoromethyl-1,2,3,4,4a,9,9a,10 (H)-octahydroacridin-10-ylcarbonylimidazol,
Nr. 80 7-Trifluoromethyl-1,2,3,4,4a,9,9a,10 (H)-octahydroacridin-10-ylcarbonylimidazol,
Nr. 81 6-Chloro-5-methyl-1,2,3,4,4a,9,9a,10 (H)-octahydroacridin-10-ylcarbonylimidazol,
Nr. 82 5,6-Dichloro-1,2,3,4,4a,9,9a,10 (H)-octahydroacridin-10-ylcarbonylimidazol,
Nr. 83 5,7-Dichloro-1,2,3,4,4a,9,9a,10 (H)-octahydroacridin-10-ylcarbonylimidazol,
Nr. 84 4-Chloro-2,2,7,8-tetramethyl-1 (H),2-dihydrochinolin-1-ylcarbonylimidazol,
Nr. 85 2,2-Dimethyl-4,7,8-trichloro-1 (H),2-dihydrochinolin-1-ylcarbonylimidazol,
Nr. 86 4,6-Dichloro-2,2-dimethyl-1 (H),2-dihydrochinolin-1-ylcarbonylimidazol,
Nr. 87 4-Chloro-1 (H),2-dihydrochinolin-1-ylcarbonylimidazol,
Nr. 88 7,8-Dimethyl-2-ethyl-1 (H),2,3,4-tetrahydrochinolin-1-ylcarbonylimidazol,
Nr. 89 7,8-Dimethyl-2-isopropyl-1 (H),2,3,4-tetrahydrochinolin-1-ylcarbonylimidazol,
Nr. 90 2,3,7,8-Tetramethyl-1 (H),2,3,4-tetrahydrochinolin-1-ylcarbonylimidazol,
$n_D^{20}$ 1,5684,
Nr. 91 8-Chloro-2,2-dimethyl-1 (H),2-dihydrochinolin-1-ylcarbonylimidazol,
Nr. 92 6-Chloro-2,2,8-trimethyl-1 (H),2-dihydrochinolin-1-ylcarbonylimidazol,
Nr. 93 6-Ethoxy-2,2-dimethyl-1 (H),2-dihydrochinolin-1-ylcarbonylimidazol,
Nr. 94 6-Ethoxy-2,2,4-trimethyl-1 (H),2-dihydrochinolin-1-ylcarbonylimidazol,
Nr. 95 8-Chloro-2,2-dimethyl-1 (H),2,3,4-tetrahydrochinolin-1-ylcarbonylimidazol,
Nr. 96 6,8-Dichloro-2,2-dimethyl-1 (H),2,3,4-tetrahydrochinolin-1-ylcarbonylimidazol,
Nr. 97 6-Chloro-2,2,8-trimethyl-1 (H),2,3,4-tetrahydrochinolin-1-ylcarbonylimidazol,
Nr. 98 6-Ethoxy-2,2-dimethyl-1 (H),2,3,4-tetrahydrochinolin-1-ylcarbonylimidazol
Nr. 99 6-Ethoxy-2,2,4-trimethyl-1 (H),2,3,4-tetrahydrochinolin-1-ylcarbonylimidazol,
Schmp. 91-95°C.
No.100 6-Brom-2,2-dimethyl-1 (H),2,3,4-tetrahydrochinolin 1-ylcarbonylimidazol
No.101 2,2-Dimethyl-6-isopropyl-1 (H),2,3,4-tetrahydrochinolin-1-ylcarbonylimidazol
No.102 2,2-Dimethyl-6-trifluoromethyl-1 (H)-,2,3,4-tetrahydrochinolin-1-ylcarbonylimidazol
No.103 2,2-Dimethyl-6-trifluoromethoxy-1 (H)-,2,3,4-tetrahydrochinolin-1-ylcarbonylimidazol
No.104 2,2-Dimethyl-6-trifluoromethylthio-1 (H),2,3,4-tetrahydrochinolin-1-ylcarbonylimidazol
No.105 6-Chlor-2-methyl-1 (H),2,3,4-tetrahydrochinolin 1-ylcarbonylimidazol
No.106 2,6-Dimethyl-1 (H),2,3,4-tetrahydrochinolin-1-yl carbonylimidazol
No.107 2-Methyl-6-trifluoromethyl-1 (H),2,3,4-tetrahydrochinolin-1-ylcarbonylimidazol
No.108 2-Isopropyl-6-trifluoromethyl-1 (H)-,2,3,4-tetrahydrochinolin-1-ylcarbonylimidazol
No.109 2,6-Dimethyl-2-isopropyl-1 (H),2,3,4-tetrahydrochinolin-1-ylcarbonylimidazol
No.110 6-Chlor-2-isopropyl-2-methyl-1 (H)-,2,3,4-tetrahydrochinolin-1-ylcarbonylimidazol
No.111 6-Chlor-2,2,4-trimethyl-1 (H),2,3,4-tetrahydrochinolin-1-ylcarbonylimidazol
No.112 2,2,4,6-Tetramethyl-1 (H),2,3,4-tetrahydro chinolin-1-ylcarbonylimidazol
No.113 6-Fluor-2,2,4-trimethyl-1 (H),2,3,4-tetrahydrochinolin-1-ylcarbonylimidazol
No.114 6-Brom-2,2,4-trimethyl-1 (H),2,3,4-tetrahydro chinolin-1-ylcarbonylimidazol
No.115 6-Trifluormethyl-2,2,4-trimethyl-1 (H)-,2,3,4-tetrahydrochinolin-1-ylcarbonylimidazol
No.116 6-Trifluormethoxy-2,2,4-trimethyl-1 (H)-,2,3,4-tetrahydrochinolin-1-ylcarbonylimidazol
No.117 2,2,6,7-Tetramethyl-1 (H),2,3,4-tetrahydro chinolin-1-ylcarbonylimidazol
No.118 6,7-Dichlor-2,2-dimethyl-1 (H),2,3,4-tetrahydrochinolin-1-ylcarbonylimidazol
No.119 7-Chlor-2,2,8-trimethyl-1 (H),2-dihydro chinolin-1-ylcarbonylimidazol
No.120 6-Brom-2,2-dimethyl-1 (H),2-dihydro chinolin-1-ylcarbonylimidazol
No.121 2,2-Dimethyl-6-isopropyl-1 (H),2-dihydro chinolin-1-ylcarbonylimidazol
No.122 2,2-Dimethyl-6-methylthio-1 (H),2-dihydro chinolin-1-ylcarbonylimidazol
No.123 2,2-Dimethyl-6-trifluoromethoxy-1 (H)-,2-dihydrochinolin-1-ylcarbonylimidazol
No.124 6-Difluor-methoxy-2,2-dimethyl-1 (H),2-dihydro chinolin-1-ylcarbonylimidazol
No.125 6-Chlor-2-methyl-1 (H),2-dihydro chinolin-1-ylcarbonylimidazol
No.126 2,6-Dimethyl-1 (H),2-dihydrochinolin-1-ylcarbonylimidazol
No.127 2-Methyl-6-trifluoromethyl-1 (H),2-dihydro chinolin-1-ylcarbonylimidazol
No.128 6-Isopropyl-2-methyl-1 (H),2-dihydro chinolin-1-ylcarbonylimidazol
No.129 2,6-Diisopropyl-1 (H),2-dihydrochinolin-1-ylcarbonylimidazol
No.130 2,6-Dimethyl-2-isopropyl-1 (H),2-dihydro chinolin-1-ylcarbonylimidazol
No.131 6-chloro-2-isopropyl-2-methyl-1 (H),2-dihydrochinolin-1-ylcarbonylimidazol
No.132 2-Isopropyl-2-methyl-6-trifluoromethyl-1 (H),2-dihydrochinolin-1-ylcarbonylimidazol
No.133 2,2,4,6-Tetramethyl-1 (H),2-dihydrochinolin-1-ylcarbonylimidazol
No.134 6-flucro-2,2,4-trimethyl-1 (H),2-dihydrochinolin-1-ylcarbonylimidazol
No.135 6-bromo-2,2,4-trimethyl-1 (H),2-dihydrochinolin-1-ylcarbonylimidazol
No.136 6-trifluoromethyl-2,2,4-trimethyl-1 (H)-,2-dihydrochinolin-1-ylcarbonylimidazol
No.137 6-trifluoromethoxy-2,2,4-trimethyl-1 (H),2-dihydrochinolin-1-ylcarbonylimidazol
No.138 6-Isopropyl-2,2,4-trimethyl-1 (H),2-dihydro chinolin-1-ylcarbonylimidazol
No.139 2,2,6,7-Tetramethyl-1 (H),2-dihydro chinolin-1-ylcarbonylimidazol
No.140 6,7-Dichlor-2,2-dimethyl-1 (H),2-dihydrochinolin-1-ylcarbonylimidazol
No.141 2,2,4,6,7-Pentamethyl-1 (H),2-dihydrochinolin-1-ylcarbonylimidazol

Beispiel 3

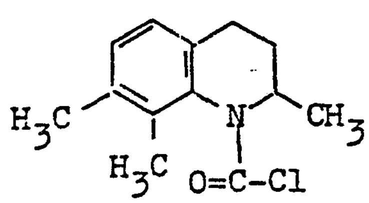

(Verbindung Nr. IV-1)

Eine Lösung von 20 ml Trichloromethylchlorameisensäureester in 100 ml Ethylacetat wurde auf 50°C bis 60°C (Bad-Temperatur) erwärmt. Zu dieser Lösung wurde dann tropfenweise unter Rühren im Laufe von 30 min eine Lösung von 9 g 2,7,8-Trimethyl-1,2,3,4-tetrahydrochinolin in 20 ml Ethylacetat hinzugefügt. Die Temperatur wurde dann langsam auf Rückfluß-Temperatur gesteigert, und das Rühren wurde 5 h fortgesetzt. Das Lösungsmittel wurde dann auf einem Dampfbad abdestilliert, und der Rückstand wurde im Hochvakuum destilliert, wonach 9 g des gewünschten 2,7,8-Trimethyl-1 (H),2,3,4-tetrahydrochinolin-1-yl-carbonylchlorids erhalten wurden. Sdp. 174-176°C/1.2 mbar (0,9 mmHg).

Verbindungen der allgemeinen Formel (IV), die nach der gleichen Arbeitsweise, wie sie in dem vorstehenden Beispiel 3 beschrieben ist, synthetisiert wurden, sind in der nachstehenden Tabelle 2 aufgeführt.

Tabelle 2

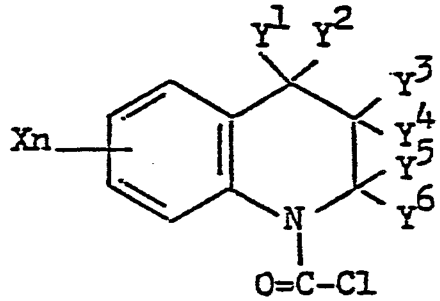

Tabelle 2 - Fortsetzung

| Verbindung. Nr. | 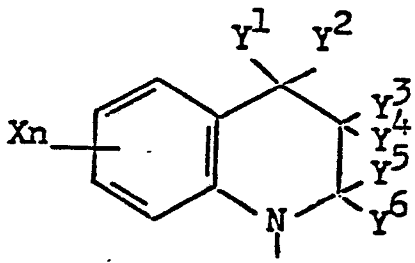 | Physikal. Konstante Schmelzpunkt (Schmp.) Siedepunkt (Sdp.) |
|---|---|---|
| IV-2 | 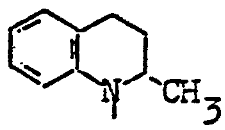 | Sdp. 127-129°C/ 0,67 mbar (0,5 mmHg) |
| IV-3 | 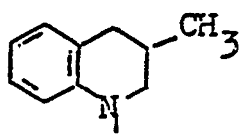 | Sdp. 120°C/ 0,67 mbar (0,5 mmHg) |
| IV-4 | 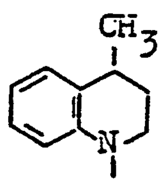 | Sdp. 137-139°C/ 0,67 mbar (0,5 mmHg) |
| IV-6 | 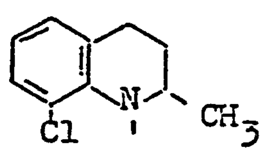 | Sdp. 157-160°C/ 0,67 mbar (0,5 mmHg) |
| IV-8 | 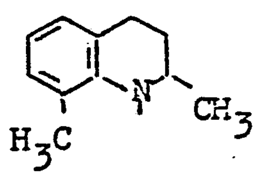 | Sdp. 136°C/ 0,67 mbar (0,5 mmHg) |
| IV-11 | 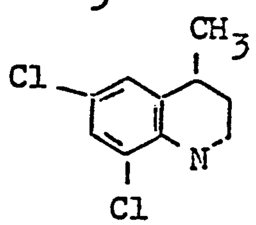 | Schmp. 104 - 107°C |

Tabelle 2 - Fortsetzung

| Verbindung Nr. | 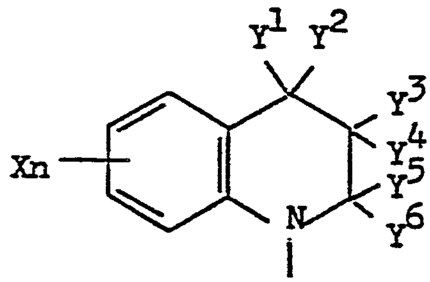 | Physikal. Konstante Schmelzpunkt (Schmp.) Siedepunkt (Sdp.) |
|---|---|---|
| IV-12 | 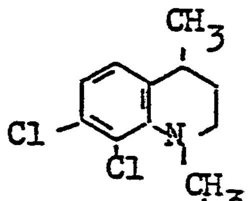 | Sdp. 165°C/ 0,4 mbar (0,3 mmHg) |
| IV-14 | 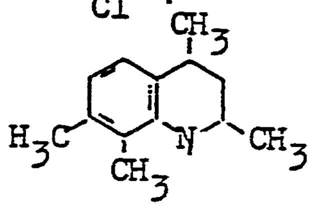 | Sdp. 137-140°C/ 0,33 mbar (0,25 mmHg) |
| IV-15 | 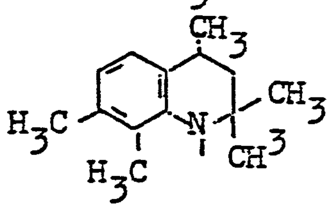 | Sdp. 140-143°C/ 0,33 mbar (0,25 mmHg) |
| IV-17 | 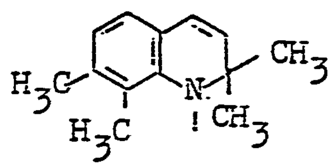 | Schmp. 103-105°C |
| IV-18 | 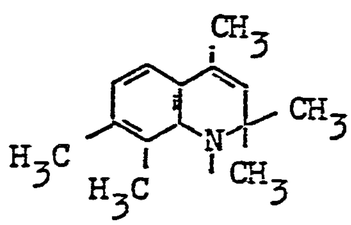 | Sdp. 138-142°C/ 0,27 mbar (0,2mmHg) |
| IV-19 | 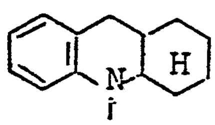 | Sdp. 175-177°C/ 0,80 mbar (0,6 mmHg) |

Tabelle 2 - Fortsetzung

| Verbindung Nr. | 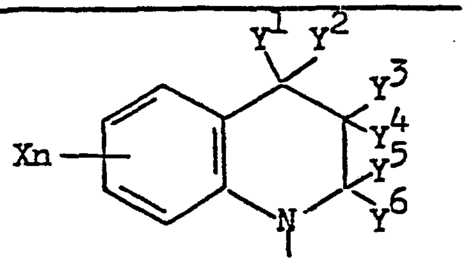 | Physikal. Konstante Schmelzpunkt (Schmp.) Siedepunkt (Sdp.) |
|---|---|---|
| IV-20 | 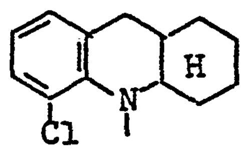 | Sdp. 200°C/ 1,6 mbar (1,2 mmHg) |
| IV-21 | 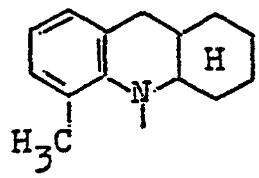 | Sdp. 166-170°C/ 0,67 mbar (0,5 mmHg ) |
| IV-22 | 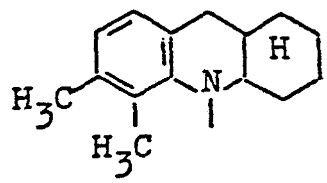 | Sdp. 175-177°C/ 0,67 mbar (0,5 mmHg ) |
| IV-47 | 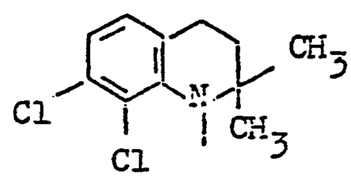 | Schmp. 80 - 83°C |
| IV-58 | 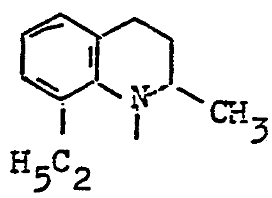 | Sdp. 172 - 175°C/ 1,33 mbar (1,0 mmHg ) |

Zusätzlich zu den im Vorstehenden beispielhaft genannten Verbindungen werden andere spezielle Beispiele für die Verbindungen der allgemeinen Formel (IV), die nach ähnlichen Arbeitsweisen wie Beispiel 3 erhalten werden, im Folgenden aufgeführt.

Nr. IV-7 8-Fluoro-2-methyl-1 (H),2,3,4-tetrahydrochinolin-1-ylcarbonylchlorid,

Nr. IV-9 8-Ethoxy-2-methyl-1 (H),2,3,4-tetrahydrochinolin-1-ylcarbonylchlorid,

Nr. IV-10 7,8-Dichloro-3-methyl-1 (H),2,3,4-tetrahydrochinolin-1-ylcarbonylchlorid,

Nr. IV-13 2,2,7,8-Tetramethyl-1 (H),2,3,4-tetrahydrochinolin-1-ylcarbonylchlorid,

Nr. IV-16 7,8-Dichloro-1 (H),2-dihydrochinolin-1-ylcarbonylchlorid,

Nr. IV-23 2-Ethyl-1 (H),2,3,4-tetrahydrochinolin-1-ylcarbonylchlorid,

Nr. IV-24 2-Propyl-1 (H),2,3,4-tetrahydrochinolin-1-ylcarbonylchlorid,

Nr. IV-25 2-Isopropyl-1 (H),2,3,4-tetrahydrochinolin-1-ylcarbonylchlorid,

Nr. IV-26 2,2-Dimethyl-1 (H),2,3,4-tetrahydrochinolin-1-ylcarbonylchlorid,

Nr. IV-27 2,2-Diethyl-1 (H),2,3,4-tetrahydrochinolin-1-ylcarbonylchlorid,
Nr. IV-28 2,2,4-Trimethyl-1 (H),2,3,4-tetrahydrochinolin-1-ylcarbonylchlorid,
Nr. IV-29 8-Chloro-2-ethyl-1 (H),2,3,4-tetrahydrochinolin-1-ylcarbonylchlorid,
Nr. IV-30 6-Chloro-2,2-dimethyl-1 (H),2,3,4-tetrahydrochinolin-1-ylcarbonylchlorid,
Nr. IV-31 8-Chloro-2,2,4-trimethyl-1 (H),2,3,4-tetrahydrochinolin-1-ylcarbonylchlorid,
Nr. IV-32 8-Bromo-2,2-dimethyl-1 (H),2,3,4-tetrahydrochinolin-1-ylcarbonylchlorid,
Nr. IV-33 2-Isopropyl-6-methyl-1 (H),2,3,4-tetrahydrochinolin-1-ylcarbonylchlorid,
Nr. IV-34 2,2,6-Trimethyl-1 (H),2,3,4-tetrahydrochinolin-1-ylcarbonylchlorid,
Nr. IV-35 2,2,8-Trimethyl-1 (H),2,3,4-tetrahydrochinolin-1-ylcarbonylchlorid,
Nr. IV-36 2,2-Dimethyl-8-methoxy-1 (H),2,3,4-tetrahydrochinolin-1-ylcarbonylchlorid,
Nr. IV-37 8-Methoxy-2,2,4-trimethyl-1 (H)-,2,3,4-tetrahydrochinolin-1-ylcarbonylchlorid,
Nr. IV-38 2,2-Dimethyl-8-isopropoxy-1 (H)-,2,3,4-tetrahydrochinolin-1-ylcarbonylchlorid,
Nr. IV-39 2,2-Dimethyl-8-methylthio-1 (H)-,2,3,4-tetrahydrochinolin-1-ylcarbonylchlorid,
Nr. IV-40 2,2-Dimethyl-8-phenoxy-1 (H),2,3,4-tetrahydrochinolin-1-ylcarbonylchlorid,
Nr. IV-41 2-Methyl-8-phenylthio-1 (H),2,3,4-tetrahydrochinolin-1-ylcarbonylchlorid,
Nr. IV-42 2-Methyl-8-nitro-1 (H),2,3,4-tetrahydrochinolin-1-ylcarbonylchlorid,
Nr. IV-43 2-Methyl-8-trifluoromethyl-1 (H)-,2,3,4-tetrahydrochinolin-1-ylcarbonylchlorid,
Nr. IV-44 2,2-Dimethyl-8-trifluoromethyl-1 (H)-,2,3,4-tetrahydrochinolin-1-ylcarbonylchlorid,
Nr. IV-45 8-Cyano-2,2-dimethyl-1 (H),2,3,4-tetrahydrochinolin-1-ylcarbonylchlorid,
Nr. IV-46 6,8-Dichloro-2,2,4-trimethyl-1 (H)-,2,3,4-tetrahydrochinolin-1-ylcarbonylchlorid,
Nr. IV-48 7,8-Dichloro-2,2,4-trimethyl-1 (H)-,2,3,4-tetrahydrochinolin-1-ylcarbonylchlorid,
Nr. IV-49 2,2,6,8-Tetramethyl-1 (H),2,3,4-tetrahydrochinolin-1-ylcarbonylchlorid,
Nr. IV-50 7-Chloro-2,8-dimethyl-1 (H),2,3,4-tetrahydrochinolin-1-ylcarbonylchlorid,
Nr. IV-51 7-Chloro-2-ethyl-8-methyl-1 (H)-,2,3,4-tetrahydrochinolin-1-ylcarbonylchlorid,
Nr. IV-52 8-Chloro-2,7-dimethyl-1 (H),2,3,4-tetrahydrochinolin-1-ylcarbonylchlorid,
Nr. IV-53 7-Chloro-2,2,8-trimethyl-1 (H),2,3,4-tetrahydrochinolin-1-ylcarbonylchlorid,
Nr. IV-54 8-Chloro-2,2,7-trimethyl-1 (H),2,3,4-tetrahydrochinolin-1-ylcarbonylchlorid,
Nr. IV-55 2,2-Dimethyl-6-fluoro-1 (H),2,3,4-tetrahydrochinolin-1-ylcarbonylchlorid,
Nr. IV-56 2,2-Dimethyl-6-methoxy-1 (H),2,3,4-tetrahydrochinolin-1-ylcarbonylchlorid,
Nr. IV-57 2,2-Dimethyl-6-nitro-1 (H),2,3,4-tetrahydrochinolin-1-ylcarbonylchlorid,
Nr. IV-58 8-Ethyl-2-methyl-1 (H),2,3,4-tetrahydrochinolin-1-ylcarbonylchlorid,
Nr. IV-59 2,8-Dimethyl-7-fluoro-1 (H),2,3,4-tetrahydrochinolin-1-ylcarbonylchlorid,
Nr. IV-60 2,2-Dimethyl-1 (H),2-dihydrochinolin-1-ylcarbonylchlorid,
Nr. IV-61 2,2,4-Trimethyl-1 (H),2-dihydrochinolin-1-ylcarbonylchlorid,
Nr. IV-62 6-Chloro-2,2-dimethyl-1 (H),2-dihydrochinolin-1-ylcarbonylchlorid,
Nr. IV-63 2,2-Dimethyl-6-fluoro-1 (H),2-dihydrochinolin-1-ylcarbonylchlorid,
Nr. IV-64 2,2,6-Trimethyl-1 (H),2-dihydrochinolin-1-ylcarbonylchlorid,
Nr. IV-65 6-Chloro-2,2,4-trimethyl-1 (H),2-dihydrochinolin-1-ylcarbonylchlorid,
Nr. IV-66 4-Chloro-2,2-dimethyl-1 (H),2-dihydrochinolin-1-ylcarbonylchlorid,
Nr. IV-67 4,6-Dichloro-2,2-dimethyl-1 (H),2-dihydrochinolin-1-ylcarbonylchlorid,
Nr. IV-68 4-Chloro-2,2,6-trimethyl-1 (H),2-dihydrochinolin-1-ylcarbonylchlorid,
Nr. IV-69 6,8-Dichloro-4-methyl-1 (H),2-dihydrochinolin-1-ylcarbonylchlorid,
Nr. IV-70 6,8-Dichloro-2,2-dimethyl-1 (H),2-dihydrochinolin-1-ylcarbonylchlorid,
Nr. IV-71 2,2-Dimethyl-6-methoxy-1 (H),2-dihydrochinolin-1-ylcarbonylchlorid,
Nr. IV-72 2,2-Dimethyl-6-trifluoromethyl-1 (H)-,2-dihydrochinolin-1-ylcarbonylchlorid,
Nr. IV-73 2,2,6,8-Tetramethyl-1 (H),2-dihydrochinolin-1-ylcarbonylchlorid,
Nr. IV-74 7,8-Dichloro-2,2-dimethyl-1 (H),2-dihydrochinolin-1-ylcarbonylchlorid,
Nr. IV-75 7-Chloro-2,2,8-trimethyl-1 (H),2-dihydrochinolin-1-ylcarbonylchlorid,
Nr. IV-76 7-Chloro-1,2,3,4,4a,9,9a,10 (H)-octahydroacridin-10-yl-carbonylchlorid,
Nr. IV-77 7-Methyl-1,2,3,4,4a,9,9a,10 (H)-octahydroacridin-10-yl-carbonylchlorid,
Nr. IV-78 7-Methoxy-1,2,3,4,4a,9,9a,10 (H)-octahydroacridin-10-yl-carbonylchlorid,
Nr. IV-79 5-Trifluoromethyl-1,2,3,4,4a,9,9a,10 (H)-octahydroacridin-10-yl-carbonylchlorid,
Nr. IV-80 7-Trifluoromethyl-1,2,3,4,4a,9,9a,10 (H)-octahydroacridin-10-yl-carbonylchlorid,
Nr. IV-81 6-Chloro-5-methyl-1,2,3,4,4a,9,9a,10 (H)-octahydroacridin-10-yl-carbonylchlorid,
Nr. IV-82 5,6-Dichloro-1,2,3,4,4a,9,9a,10 (H)-octahydroacridin-10-yl-carbonylchlorid,
Nr. IV-83 5,7-Dichloro-1,2,3,4,4a,9,9a,10 (H)-octahydroacridin-10-yl-carbonylchlorid,

Nr. IV-84 4-Chloro-2,2,7,8-tetramethyl-1 (H),2-dihydrochinolin-1-ylcarbonylchlorid,
Nr. IV-85 2,2-Dimethyl-4,7,8-trichloro-1 (H),2-dihydrochinolin-1-ylcarbonylchlorid,
Nr. IV-86 4-Chloro-1 (H),2-dihydrochinolin-1-ylcarbonylchlorid,
Nr. IV-87 2,3,7,8-Tetramethyl-1 (H),2,3,4-tetrahydrochinolin-1-ylcarbonylchlorid,
Nr. IV-88 7,8-Dimethyl-2-ethyl-1 (H),2,3,4-tetrahydrochinolin-1-ylcarbonylchlorid,
Nr. IV-89 7,8-Dimethyl-2-isopropyl-1 (H),2,3,4-tetrahydrochinolin-1-ylcarbonylchlorid,
Nr. IV-90 8-Chloro-2,2-dimethyl-1 (H),2-dihydrochinolin-1-ylcarbonylchlorid,
Nr. IV-91 6-Chloro-2,2,8-trimethyl-1 (H),2-dihydrochinolin-1-ylcarbonylchlorid,
Nr. IV-92 6-Ethoxy-2,2-dimethyl-1 (H),2-dihydrochinolin-1-ylcarbonylchlorid,
Nr. IV-93 6-Ethoxy-2,2,4-trimethyl-1 (H),2-dihydrochinolin-1-ylcarbonylchlorid,
Nr. IV-94 6,8-Dichloro-2,2-dimethyl-1 (H),2,3,4-tetrahydrochinolin-1-ylcarbonylchlorid,
Nr. IV-95 6-Chloro-2,2,8-trimethyl-1 (H),2,3,4-tetrahydrochinolin-1-ylcarbonylchlorid,
Nr. IV-96 6-Ethoxy-2,2-dimethyl-1 (H),2,3,4-tetrahydrochinolin-1-ylcarbonylchlorid und
Nr. IV-97 6-Ethoxy-2,2,4-trimethyl-1 (H),2,3,4-tetrahydrochinolin-1-ylcarbonylchlorid,
No.IV-98 6-Bromo-2,2-dimethyl-1 (H),2,3,4-tetrahydrochinolin-1--ylcarbonylchlorid
No.IV-99 2,2-Dimethyl-6-isopropyl-1 (H),2,3,4-tetrahydrochinolin-1-ylcarbonylchlorid
No.IV-100 2,2-Dimethyl-6-trifluoromethyl-1 (H)-,2,3,4-tetrahydrochinolin-1-ylcarbonylchlorid
No.IV-101 2,2-Dimethyl-6-trifluoromethoxy-1 (H),2,3,4-tetrahydrochinolin-1-ylcarbonylchlorid
No.IV-102 2,2-Dimethyl-6-trifluoromethylthio-1 (H),2,3,4-tetrahydrochinolin-1-ylcarbonylchlorid
No.IV-103 6-Chloro-2-methyl-1 (H),2,3,4-tetrahydrochinolin-1-ylcarbonylchlorid
No.IV-104 2,6-Dimethyl-1 (H),2,3,4-tetrahydrochinolin-1-ylcarbonylchlorid
No.IV-105 2-Methyl-6-trifluoromethyl-1 (H)-,2,3,4-tetrahydrochinolin-1-ylcarbonylchlorid
No.IV-106 2-Isopropyl-6-trifluoromethyl-1 (H)-,2,3,4-tetrahydrochinolin-1-ylcarbonylchlorid
No.IV-107 2,6-Dimethyl-2-isopropyl-1 (H),2,3,4-tetrahydrochinolin-1-ylcarbonylchlorid
No.IV-108 6-Chlor-2-isopropyl-2-methyl-1 (H)-,2,3,4-tetrahydrochinolin-1-ylcarbonylchlorid
No.IV-109 6-Chlor-2,2,4-trimethyl-1 (H),2,3,4-tetrahydrochinolin-1-ylcarbonylchlorid
No.IV-110 2,2,4,6-Tetramethyl-1 (H),2,3,4-tetrahydrochinolin-1-ylcarbonylchlorid
No.IV-111 6-Fluor-2,2,4-trimethyl-1 (H),2,3,4-tetrahydrochinolin-1-ylcarbonylchlorid
No.IV-112 6-Brom-2,2,4-trimethyl-1 (H),2,3,4-tetrahydrochinolin-1-ylcarbonylchlorid
No.IV-113 6-Trifluoromethyl-2,2,4-trimethyl-1 (H),2,3,4-tetrahydrochinolin-1-ylcarbonylchlorid
No.IV-114 6-Difluoromethoxy-2,2,4-trimethyl-1 (H),2,3,4-tetrahydrochinolin-1-ylcarbonylchlorid
No.IV-115 2,2,6,7-Tetramethyl-1 (H),2,3,4-tetrahydrochinolin-1-ylcarbonylchlorid
No.IV-116 6,7-Dichloro-2,2-dimethyl-1 (H)-,2,3,4-tetrahydrochinolin-1-ylcarbonylchlorid
No.IV-117 7-Chlor-2,2,8-trimethyl-1 (H),2-dihydro chinolin-1-ylcarbonylchlorid
No.IV-118 6-Brom-2,2-dimethyl-1 (H),2-dihydro chinolin-1-ylcarbonylchlorid
No.IV-119 2,2-Dimethyl-6-isopropyl-1 (H),2-dihydro chinolin-1-ylcarbonylchlorid
No.IV-120 2,2-Dimethyl-6-methylthio-1 (H),2-dihydro chinolin-1-ylcarbonylchlorid
No.IV-121 2,2-Dimethyl-6-trifluoromethoxy-1 (H),2-dihydrochinolin-1-ylcarbonylchlorid
No.IV-122 6-Difluoromethoxy-2,2-dimethyl-1 -(H),2-dihydrochinolin-1-ylcarbonylchlorid
No.IV-123 6-Chlor-2-methyl-1 (H),2-dihydro chinolin-1-ylcarbonylchlorid
No.IV-124 2,6-Dimethyl-1 (H),2-dihydro chinolin-1-ylcarbonylchlorid
No.IV-125 2-Methyl-6-trifluoromethyl-1 (H),2-dihydro chinolin-1-ylcarbonylchlorid
No.IV-126 6-Isopropyl-2-methyl-1 (H),2-dihydro chinolin-1-ylcarbonylchlorid
No.IV-127 2,6-Diisopropyl-1 (H),2-dihydro chinolin-1-ylcarbonylchlorid
No.IV-128 2,6-Dimethyl-2-isopropyl-1 (H),2-dihydrochinolin-1-ylcarbonylchlorid
No.IV-129 6-Chlor-2-isopropyl-2-methyl-1 (H)-,2-dihydrochinolin-1-ylcarbonylchlorid
No.IV-130 2-Isopropyl-2-methyl-6-trifluoromethyl-1 (H),2-dihydrochinolin-1-ylcarbonylchlorid
No.IV-131 2,2,4,6-Tetramethyl-1 (H),2-dihydro chinolin-1-ylcarbonylchlorid
No.IV-132 6-Fluor-2,2,4-trimethyl-1 (H),2-dihydro chinolin-1-ylcarbonylchlorid
No.IV-133 6-Brom-2,2,4-trimethyl-1 (H),2-dihydro chinolin-1-ylcarbonylchlorid
No.IV-134 6-Trifluormethyl-2,2,4-trimethyl-1 -(H),2-dihydrochinolin-1-ylcarbonylchlorid
No.IV-135 6-Trifluor methoxy-2,2,4-trimethyl-1 (H),2-dihydrochinolin-1-ylcarbonylchlorid
No.IV-136 6-Isopropyl-2,2,4-trimethyl-1 (H),2-dihydro chinolin-1-ylcarbonylchlorid
No.IV-137 2,2,6,7-Tetramethyl-1 (H),2-dihydro chinolin-ylcarbonylchlorid
No.IV-138 6,7-Dichlor-2,2-dimethyl-1 (H),2-dihydrochinolin 1-ylcarbonylchlorid
No.IV-139 2,2,4,6,7-Pentamethyl-1 (H),2-dihydrochinolin-1-ylcarbonylchlorid

Verwendungsbeispiele

Kontroll-Verbindung Z-1

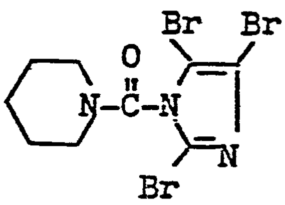

(die in der JP-Patentveröffentlichung 17 748/1968 offenbarte Verbindung)

Beispiel 4

Test zur Bestimmung der Wirksamkeit gegen Reisfeld-Unkräuter bei Behandlung von Stengeln, Blättern und Boden im überfluteten Zustand

Topf-Test

Herstellung der aktiven Verbindung:

Träger: 5 Gew.-Teile Aceton

Emulgator: 1 Gew.-Teil Benzyloxypolyglycolether

Die aktive Verbindung wird als Emulsion durch Vermischen von 1 Gew.-Teil der aktiven Verbindung mit den oben angegebenen Mengen Träger und Emulgator erhalten. Die vorgeschriebene Dosis der Mischung der aktiven Verbindung wird dann durch Verdünnen mit Wasser eingestellt.

Test-Verfahren

Wagner-Töpfe (2 $dm^2$) wurden mit wasserhaltigem Reis-Kulturboden gefüllt, und zwei Reis-Setzlinge (Varietät: Kinmaze) im 2-bis 3-Blattstadium wurden in jeden Topf umgepflanzt. In die Töpfe wurden Samen von Echinochloa crus-galli, Cyperus microiria, Monochoria vaginalis, Scirpus juncoides und breitblättrigen Unkräutern, kleine Stücke von Eleocharis acicularis und Knollen von Cyperus serotinus eingesät bzw. eingesetzt. Die Erde in den Töpfen wurde dann in feuchtem Zustand gehalten. Als Echinochloa crus-galli etwa bis zum 2-Blatt-Stadium gewachsen war (etwa 7 bis 9 Tage nach der Einsaat), wurden die Töpfe mit Wasser bis zu einer Tiefe von etwa 6 cm gefüllt, und eine vorher festgelegte Menge der Verbindung der vorliegenden Erfindung wurde in Form einer Emulsion mittels einer Pipette zur Behandlung jedes Topfes zugegeben. Nach der Behandlung wurde das Wasser im Laufe von zwei Tagen mit einer Geschwindigkeit von 2 bis 3 cm pro Tag aus den Töpfen heraussickern gelassen. Danach wurde die Wassertiefe in den Töpfen bei etwa 3 cm gehalten. In der vierten Woche nach der Behandlung mit der Chemikalie wurden die herbizide Wirkung und der Grad der Phytotoxizität mit Hilfe einer Skala von 0 bis 5 nach dem folgenden Maßstab bewertet.

Bewertung der herbiziden Wirkung (Herbizid-Rate im Vergleich mit der einer unbehandelten Fläche)

| | |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

Bewertung der Phytotoxizität gegen bewässerte Reis-Pflanzen (Phytotoxizitätsrate im Vergleich mit der einer unbehandelten Fläche)

| | | |
|---|---|---|
| 5 | wenigstens 90 % | (Ausrottung) |
| 4 | wenigstens 50 %, | jedoch weniger als 90 % |
| 3 | wenigstens 30 %, | jedoch weniger als 50 % |
| 2 | wenigstens 10 %, | jedoch weniger als 30 % |
| 1 | mehr als 0 %, | jedoch weniger als 10 % |
| 0 | 0 % | (keine Phytotoxizität) |

Die Testergebnisse sind in Tabelle 3 aufgeführt, wobei als typische Beispiele die folgenden Unkräuter benutzt wurden, die der Kürze wegen mit A, B, C, D, E, F und G wie nachstehend angegeben bezeichnet wurden.

A: Echinochloa crus-galli
B: Eleocharis acicularis
C: Cyperus microiria
D: Scirpus juncoides
E: Monochoria vaginalis
F: Breitblättrige Unkräuter (Lindernia procumbens, Rotala indica, Elatine triandra etc.)
G: Cyperus serotinus

Tabelle 3

| Verbindung Nr. | Wirkstoff-Menge kg/ha | Herbizide Wirkung Unkräuter A | B | C | D | E | F | G | Phytotoxizität gegen Reispflanzen |
|---|---|---|---|---|---|---|---|---|---|
| 1 | 1,0 | 5 | 5 | 5 | 5 | 5 | 5 | 5 | 0 |
| 10 | 1,0 | 5 | 5 | 5 | 5 | 5 | 5 | 5 | 0 |
| 12 | 1,0 | 5 | 5 | 5 | 5 | 5 | 5 | 5 | 0 |
| 13 | 1,0 | 5 | 5 | 5 | 5 | 5 | 5 | 5 | 0 |
| 18 | 1,0 | 5 | 5 | 5 | 5 | 5 | 5 | 5 | 0 |
| Kontrolle Z-1 | 2,0 | 0 | 0 | 1 | 0 | 1 | 2 | 0 | 0 |

Beispiel 5

Test zur Bestimmung der Wirksamkeit der Bekämpfung von Botrytis cinerea bei Buschbohnen.

Herstellung der Test-Chemikalie

Zu testende Verbindung: 30 Teile

Organisches Lösungsmittel (Xylol): 55 Teile

Emulgator: Polyoxyethylenalkylphenylether 8 Teile

Calciumalkylbenzolsulfonat 7 Teile

Die vorgeschriebene Dosis der wie vorstehend erhaltenen Emulsion wird dann durch Verdünnen mit Wasser eingestellt.

Test-Verfahren

In unlasierten 9 cm-Töpfen angezogene Buschbohnen (Sorte: Shin-edogawa) im Stadium der ersten Blatt-Bildung wurden mit den Test-Verbindungen in Form einer Emulsion unter Verwendung einer Spritzpistole besprüht. Einen Tag nach dem Aufbringen der Emulsion wurde eine pathogenhaltige Agar-Scheibe, die vorher durch 2-tägiges Kultivieren von Botrytis cinerea in einem PDA-Medium bei 20°C und anschließendes Ausstanzen der Spitze der Kolonie mit einem Korkbohrer von 5 mm Durchmesser hergestellt worden war, in die Mitte eines Blattes gelegt und dort belassen, während das Blatt in einer Feuchtigkeitskammer bei 20°C gehalten wurde. Zwei Tage nach der Inokulierung wurde der Durchmesser einer Läsion gemessen, und die Bekämpfungswirkung wurde berechnet.

$$\text{Bekämpfungs-Index (\%)} = \frac{\text{Durchmesser der Läsion der unbehandelten Fläche} - \text{Durchmesser der Läsion der behandelten Fläche}}{\text{Durchmesser der Läsion der unbehandelten Fläche}} \times 100$$

In diesem Test zeigten die Verbindungen Nr. 13, 14 und 17 beispielsweise einen Bekämpfungs-Index von 100 % bei einer Konzentration der aktiven Komponente von 500 ppm, während der Bekämpfungs-Index der Kontroll-Verbindung Z-1 0 betrug.

Beispiel 6

Test zur Bestimmung der Wirksamkeit der Bekämpfung der Braunfäule der Tomate:

Jede der Test-Verbindungen in Form einer Emulsion, die gemäß Beispiel 5 hergestellt worden war, wurde auf in unglasierten 9 cm-Töpfen gezogene Tomaten (Sorte: "Kurihara") mit einer Sprühpistole aufgesprüht. Einen Tag später wurden die Pflanzen mit einer Sporen-Suspension des bezeichneten Pathogens inokuliert, und die Töpfe wurden über Nacht in einem Raum mit einer konstanten Temperatur von 22°C und einer Luftfeuchtigkeit oberhalb von 90 % stehen gelassen. Fünf Tage später wurde der Grad der Erkrankung durch das Verhältnis der Läsionen zeigenden Flächen bestimmt, und der Bekämpfungsindex wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung der unbehandelten Fläche} - \text{Grad der Erkrankung der behandelten Fläche}}{\text{Grad der Erkrankung der unbehandelten Fläche}} \times 100$$

Die Verbindungen Nr. 2 und 11 zeigten eine Bekämpfungs-Wirkung von 100 % bei einer Konzentration der aktiven Komponente von 500 ppm, während die oben bezeichnete Kontroll-Verbindung Z-1 einen Bekämpfungs-Wert von 0 % zeigte.

Beispiel 7

Test zur Bestimmung der Wirksamkeit gegen Cochliobolus miyabeanus bei Besprühen des Stengel und Blätter der Pflanze:

Reispflanzen (Sorte: Kusabue) wurden in unglasierten Töpfen mit einem Durchmesser von 12 cm gezogen, und im 3-bis 4-Blatt-Stadium wurde eine Test-Verbindung, die nach Beispiel 5 hergestellt und auf die vorgeschriebene Konzentration verdünnt worden war, in einer Menge von 50 ml pro jeweils drei Töpfe aufgesprüht. Am nächsten Tag wurden die Töpfe durch zweimaliges Besprühen mit einer Suspension künstlich kultivierter Sporen von Cochliobolus miyabeanus inokuliert. Die Töpfe wurden dann zur Induzierung der Infektion in einer feuchten Kammer mit einer relativen Luftfeuchtigkeit von 100% und einer Temperatur von 25°C gehalten. Sieben Tage nach der Inokulierung wurde der Grad der Erkrankung pro Topf entsprechend der nachstehenden Skala von 0 bis 5 bewertet, und der Bekämpfungs-Index wurde berechnet.

| Grad der Erkrankung | Ausmaß der Erkrankung |
|---|---|
| 0 | Keine Erkrankung |
| 1 | geringfügig |
| 2 | schwach |
| 3 | mittel |
| 4 | stark |
| 5 | sehr stark |

$$\text{Bekämpfungs-Index (\%)} = \frac{\text{Grad der Erkrankung der unbehandelten Fläche} - \text{Grad der Erkrankung der behandelten Fläche}}{\text{Grad der Erkrankung der unbehandelten Fläche}} \times 100$$

In diesem Test bildeten jeweils drei Töpfe eine Parzelle. Die Verbindungen Nr. 6, 7, 13, 17 und 20 zeigten einen Bekämpfungs-Index von 100 % bei einer Konzentration der aktiven Komponente von 500 ppm, während der Bekämpfungs-Index der oben bezeichneten Kontroll-Verbindung Z-1 0 betrug.

## Ansprüche

1. Carbamoylimidazole der Formel (I)

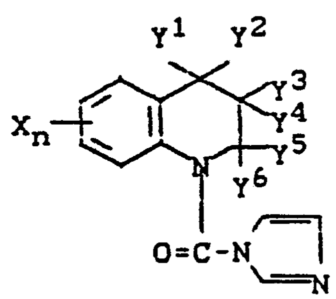

(I)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, halogensubstituiertes Niederalkylsulfonyl, halogensubstituiertes Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$, und $Y^6$ jeweils Wasserstoff, Niederalkyl, Halogen oder Phenyl sind,

wobei $Y^2$ und $Y^3$ zusammen genommen eine Einfachbindung bilden können, so daß in dem heterocyclischen Ring eine Doppelbindung gebildet wird, oder $Y^4$ und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind.

2. Carbamoylimidazole der Formel (I) nach Anspruch 1, dadurch gekennzeichnet, daß

X Fluor, Chlor, Brom, Alkyl mit 1 bis 4 Kohlenstoff-Atomen, Alkoxy mit 1 bis 4 Kohlenstoff-Atomen, Alkylthio mit 1 bis 4 Kohlenstoff-Atomen, fluor-und/oder chlorsubstituiertes Alkyl mit 1 bis 4 Kohlenstoff-Atomen, fluor-und/oder chlorsubstituiertes Alkoxy mit 1 bis 4 Kohlenstoff-Atomen, fluor-und/oder chlorsubstituiertes Alkylthio mit 1 bis 4 Kohlenstoff-Atomen, Nitro, Cyano, Alkylsulfonyl mit 1 bis 4 Kohlenstoff-Atomen, halogensubstituiertes Alkylsulfonyl mit 1 bis 4 Kohlenstoff-Atomen, Alkylsulfinyl mit 1 bis 4 Kohlenstoff-Atomen, Phenyl, Phenoxy oder Phenylthio ist,

n 0, 1 oder 2 ist und

$Y^1$, $Y^2$, $Y^3$, $Y^4$, $Y^5$ und $Y^6$ jeweils Wasserstoff, Alkyl mit 1 bis 4 Kohlenstoff-Atomen, Chlor oder Phenyl sind,

wobei $Y^2$ und $Y^3$ zusammen genommen eine Einfachbindung bilden können, so daß eine Doppelbindung in dem heterocyclischen Ring gebildet wird, und $Y^4$stoff, Alkyl und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind.

3. Verfahren zur Herstellung von Carbamoylimidazolen der Formel (I)

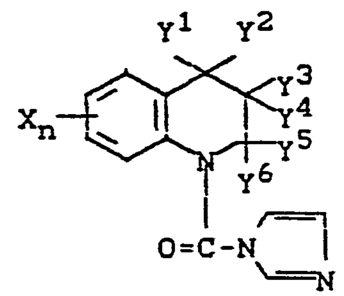

(I)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, halogensubstituiertes Niederalkylsulfonyl, halogensubstituiertes Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$ und $Y^6$ jeweils Wasserstoff, Niederalkyl, Halogen oder Phenyl sind,

wobei $Y^2$ und $Y^3$ zusammen genommen eine Einfachbindung bilden können, so daß in dem heterocyclischen Ring eine Doppelbindung gebildet wird, oder $Y^4$ und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind,

dadurch gekennzeichnet, daß

(a) die Verbindungen der Formel (II)

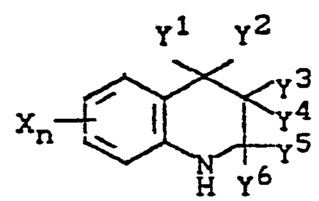

(II)

in der X, n, $Y^1$, $Y^2$, $Y^3$, $Y^4$, $Y^5$ und $Y^6$ die im Vorstehenden angegebenen Bedeutungen haben, mit N,N'-Carbonyldiimidazol der Formel (III)

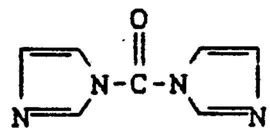

(III)

umgesetzt werden, gegebenenfalls in Gegenwart eines inerten Lösungsmittels,

oder daß

(b) die Verbindungen der Formel (IV)

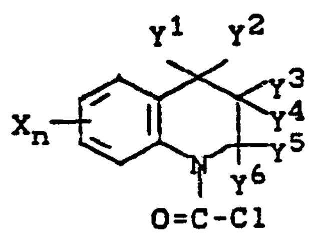

(IV)

in der X, n, $Y^1$, $Y^2$, $Y^3$, $Y^4$, $Y^5$ und $Y^6$ die im Vorstehenden angegebenen Bedeutungen haben, mit Imidazol der Formel (V)

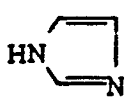

(V)

umgesetzt werden, gegebenenfalls in Gegenwart eines inerten Lösungsmittels und/oder in Gegenwart eines säurebindenden Mittels.

4. Herbizide und fungizide Mittel, dadurch gekennzeichnet, daß sie wenigstens ein Carbamoylimidazol der Formel (I) nach Ansprüchen 1 bis 3 enthalten.

5. Verfahren zur Bekämpfung von Unkräutern und Fungi, dadurch gekennzeichnet, daß man Carbamoylimidazole der Formel (I) nach Ansprüchen 1 bis 3 auf die Unkräuter und Fungi und/oder deren Umgebung einwirken läßt.

6. Verwendung von Carbamoylimidazolen der Formel (I) nach Ansprüchen 1 bis 3 zur Bekämpfung von Unkräutern und Fungi.

7. Verfahren zur Herstellung von herbiziden und fungiziden Mitteln, dadurch gekennzeichnet, daß Carbamoylimidazole der Formel (I) nach Ansprüchen 1 bis 3 mit Streckmitteln und/oder oberflächenaktiven Mitteln vermischt werden.

8. Verbindungen der Formel (IV)

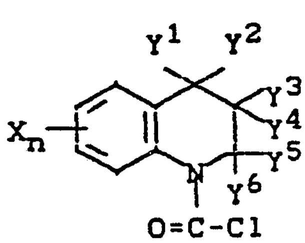

(IV)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, halogensubstituiertes Niederalkylsulfonyl, halogensubstituiertes Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$, und $Y^6$ jeweils Wasserstoff, Niederalkyl, Halogen oder Phenyl sind,

wobei $Y^2$ und $Y^3$ zusammen genommen eine Einfachbindung bilden können, so daß in dem heterocyclischen Ring eine Doppelbindung gebildet wird, oder $Y^4$ und $Y^6$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind.

9. Verfahren zur Herstellung der Verbindungen der Formel (IV)

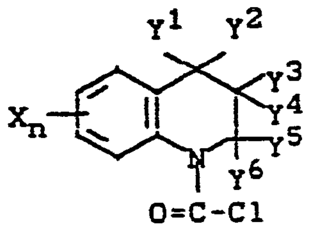

(IV)

in der

X Halogen, Niederalkyl, Niederalkoxy, Niederalkylthio, halogensubstituiertes Niederalkyl, halogensubstituiertes Niederalkoxy, halogensubstituiertes Niederalkylthio, Nitro, Cyano, Niederalkylsulfonyl, Niederalkylsulfinyl, halogensubstituiertes Niederalkylsulfonyl, halogensubstituiertes Niederalkylsulfinyl, Phenyl, Phenoxy oder Phenylthio ist,

n 0, 1, 2 oder 3 ist und

$Y^1$, $Y^2$, $Y^3$, $Y^4$, $Y^5$ und $Y^6$ jeweils Wasserstoff, Niederalkyl, Halogen oder Phenyl sind,

wobei $Y^2$ und $Y^3$ zusammen genommen eine Einfachbindung bilden können, so daß in dem heterocyclischen Ring eine Doppelbindung gebildet wird, oder $Y^4$ und $Y^5$ zusammen genommen eine Tetramethylen-Gruppe bilden können, mit der Maßgabe, daß $Y^1$, $Y^2$, $Y^3$, $Y^4$, $Y^5$ und $Y^6$ nicht alle gleichzeitig Wasserstoff sind,

dadurch gekennzeichnet, daß die Verbindungen der Formel (II)

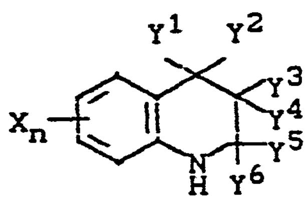

(II)

in der X, n, $Y^1$, $Y^2$, $Y^3$, $Y^4$, $Y^5$, und $Y^5$ die im Vorstehenden angegebenen Bedeutungen haben, entweder mit Phosgen der Formel $COCl_2$ oder mit Trichloromethylchlorameisensäurester der Formel $ClCOOCCl_3$ umgesetzt werden.